(19) Europäisches Patentamt
European Patent Office
Office européen des brevets

(11) **EP 2 519 259 B1**

(12) **FASCICULE DE BREVET EUROPEEN**

(45) Date de publication et mention de la délivrance du brevet:
**18.01.2017 Bulletin 2017/03**

(21) Numéro de dépôt: **10790854.3**

(22) Date de dépôt: **17.12.2010**

(51) Int Cl.:
*A61K 39/39* ^(2006.01)

(86) Numéro de dépôt international:
**PCT/EP2010/070111**

(87) Numéro de publication internationale:
**WO 2011/080126 (07.07.2011 Gazette 2011/27)**

(54) **AGONISTES DES RECEPTEURS TLR 4 ET 9 POUR PREVENIR LES COMPLICATIONS SEPTIQUES DE L'IMMUNODEPRESSION POST-TRAUMATIQUE CHEZ LES PATIENTS HOSPITALISES POUR TRAUMATISMES SEVERES**

AGONISTEN VON TLR4- UND 9-REZEPTOREN ZUR VORBEUGUNG SEPTISCHER KOMPLIKATIONEN EINER POSTTRAUMATISCHEM IMMUNSYSTEMDEPRESSION BEI PATIENTEN, DIE WEGEN EINES SCHWEREN TRAUMAS HOSPITALISIERT SIND

AGONISTS OF TLR4 AND 9 RECEPTORS FOR PREVENTING SEPTIC COMPLICATIONS OF POST-TRAUMATIC IMMUNE DEPRESSION IN PATIENTS HOSPITALIZED FOR SEVERE TRAUMA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.12.2009 FR 0906372**

(43) Date de publication de la demande:
**07.11.2012 Bulletin 2012/45**

(73) Titulaires:
• **CHU Nantes**
**44000 Nantes (FR)**
• **Université de Nantes**
**44000 Nantes (FR)**

(72) Inventeur: **ASEHNOUNE, Karim**
**F-44000 Nantes (FR)**

(74) Mandataire: **Regimbeau**
**20, rue de Chazelles**
**75847 Paris Cedex 17 (FR)**

(56) Documents cités:
**WO-A1-2005/035588     US-A1- 2006 265 767**

• **THOBE BJOERN M ET AL: "The role of MAPK in Kupffer cell toll-like receptor (TLR) 2-, TLR4-, and TLR9-mediated signaling following trauma-hemorrhage", JOURNAL OF CELLULAR PHYSIOLOGY, vol. 210, no. 3, mars 2007 (2007-03), pages 667-675, XP002588740, ISSN: 0021-9541**
• **FÖLDES GÁBOR ET AL: "Toll-like receptor modulation in cardiovascular disease: a target for intervention?", EXPERT OPINION ON INVESTIGATIONAL DRUGS AUG 2006 LNKD- PUBMED:16859390, vol. 15, no. 8, août 2006 (2006-08), pages 857-871, XP002588741, ISSN: 1744-7658**
• **LI YUEHUA ET AL: "Hemorrhagic shock augments lung endothelial cell activation: role of temporal alterations of TLR4 and TLR2", AMERICAN JOURNAL OF PHYSIOLOGY: REGULATORY, INTEGRATIVE AND COMPARATIVE PHYSIOLOGY, AMERICAN PHYSIOLOGICAL SOCIETY, US LNKD- DOI:10.1152/AJPREGU.00445.2009, vol. 297, no. 6, 1 décembre 2009 (2009-12-01), pages R1670-R1680, XP009135156, ISSN: 0363-6119**
• **PLITAS GEORGE ET AL: "Toll-like receptor 9 inhibition reduces mortality in polymicrobial sepsis", JOURNAL OF EXPERIMENTAL MEDICINE, vol. 205, no. 6, June 2008 (2008-06), pages 1277-1283, XP005517292, ISSN: 0022-1007**

- WEIGHARDT ET AL: "Increased resistance against acute polymicrobial sepsis in mice challenged with immunostimulatory CpG oligodeoxynucleotides is related to an enhanced innate effector cell response", THE JOURNAL OF IMMUNOLOGY, THE AMERICAN ASSOCIATION OF IMMUNOLOGISTS, US, vol. 165, no. 8, 1 January 2000 (2000-01-01), pages 4537-4543, XP002252726, ISSN: 0022-1767

SEQUENCE LISTING

<110> CHU NANTES UNIVERSITE DE NANTES Asehnoune , Karim

<120> AGONISTES DES RECEPTEURS TLR 4 ET 9 POUR PREVENIR LES COMPLICATIONS SEPTIQUES DE L'IMMUNODEPRESSION POST-TRAUMATIQUE CHEZ LES PATIENTS HOSPITALISES POUR TRAUMATISMES SEVERES

<130> 357288D28144

<150> FR0906372
<151> 2009-12-28

<160> 10

<170> PatentIn version 3.3

<210> 1
<211> 22
<212> DNA

<213> artificial

<220>
<223> Amorce forward TNF alpha

<400> 1
aaagggagag tggtcaggtt gc          22

<210> 2
<211> 20
<212> DNA
<213> artificial

<220>
<223> Amorce reverse TNF alpha

<400> 2
ggctggctct gtgaggaagg          20

<210> 3
<211> 20
<212> DNA
<213> artificial

<220>
<223> Amorce forward IFN beta

<400> 3
ccctatggag atgacggaga          20

<210> 4
<211> 20
<212> DNA
<213> artificial

<220>
<223> Amorce reverse IFN beta

<400> 4
ctgtctgctg gtggagttca          20

<210> 5
<211> 20
<212> DNA
<213> artificial

<220>
<223> Amorce forward IL-10

<400> 5
tggcatgagg atcagcaggg          20

<210> 6
<211> 19
<212> DNA
<213> artificial

<220>

<223> Amorce reverse IL-10

<400> 6
ggcagtccgc agctctagg        19

<210> 7
<211> 22
<212> DNA
<213> artificial

<220>
<223> Amorce forward IL-12p40

<400> 7
tgtggaatgg cgtctctgtc tg        22

<210> 8
<211> 22
<212> DNA
<213> artificial

<220>
<223> Amorce reverse IL-12p40

<400> 8
cagttcaatg ggcagggtct cc        22

<210> 9
<211> 20
<212> DNA
<213> artificial

<220>
<223> Amorce forward GAPDH

<400> 9
accacagtcc atgccatcac        20

<210> 10
<211> 18
<212> DNA
<213> artificial

<220>
<223> Amorce reverse GAPDH

<400> 10
accttgccca cagccttg        18

**Description**

La présente invention se rapporte à des compositions et méthodes utiles pour prévenir les complications septiques chez les patients hospitalisés présentant une immunodépression systémique consécutive à un ou plusieurs traumatismes sévères.

Plus précisément, la présente invention concerne une composition pharmaceutique comprenant au moins un agoniste du récepteur de type Toll (« Toll Like receptor » ou TLR) 4 (TLR4), pour son utilisation dans le traitement prophylactique des complications septiques de l'immunodépression systémique post-traumatique chez un patient ayant subi un ou plusieurs traumatismes sévères, hospitalisé, en particulier dans un service hospitalier de réanimation, dans laquelle ledit agoniste du TLR 4 est le lipide A monophosphoryle (MPLA) ou le lipide A monophosphoryle 3-O désacylaté (3D-MPLA) et ledit agoniste du TLR 9 est un oligodésoxynucléotide CpG (CpG ODN).

La présente description décrit également une composition pharmaceutique comprenant au moins un agoniste d'au moins un récepteur de type Toll (« Toll Like receptor » ou TLR) choisi parmi les TLR 4 et 9, pour son utilisation dans le traitement prophylactique des complications septiques de l'immunodépression systémique post-traumatique chez un patient ayant subi un ou plusieurs traumatismes sévères, hospitalisé, en particulier dans un service hospitalier de réanimation. De préférence, ledit agoniste du TLR 4 est le lipide A monophosphoryle (MPLA) ou le lipide A monophosphoryle 3-O désacylaté (3D-MPLA) et ledit agoniste du TLR 9 est un oligodésoxynucléotide CpG (CpG ODN).

Les traumatismes sévères sont en France et aux Etats-Unis, la première cause de mortalité au cours des trente premières années de vie et, plus globalement, la quatrième cause de décès avec respectivement plus de 160 000 et 10 000 morts chaque année (Mathers et al. 2006 ; Patton et al. 2009). Si l'on écarte la mortalité directement liée au traumatisme lui-même, la mortalité secondaire post-traumatique est essentiellement due à une immunodépression systémique (IS) spécifique, sévère et prolongée (Keel et al. 1996). La réponse physiologique à un traumatisme repose sur une activation neuro-humorale complexe. La dysrégulation de l'immunité à médiation cellulaire est responsable d'une dysrégulation immunitaire (Keel et al. 1996 ; Ditschkowski et al. 1999). Le choc hémorragique est une cause majeure de ce désordre immunitaire en raison de l'importance des lésions d'ischémie (déprivation tissulaire en oxygène pendant la phase d'ischémie (saignement)) et reperfusion (relargage massif de composés toxiques et de médiateurs de l'inflammation pendant la réanimation du blessé) (Stephan et al. 1989). De plus, les lésions tissulaires agissent de manière synergique avec l'hémorragie pour moduler de manière très spécifique la réponse immunitaire des patients traumatisés (Wichmann et al. 1998). Dans le cas bien particulier des patients hospitalisés pour traumatisme ou polytraumatisme sévère, l'IS post-traumatique qui les caractérise est directement responsable de complications septiques, généralement des infections secondaires acquises en milieu hospitalier (ou infections nosocomiales).

L'IS post-traumatique et les complications septiques associées sont donc deux cibles prioritaires si l'on veut améliorer dans l'avenir le pronostic des traumatisés sévères car le coût humain et économique des complications infectieuses post-traumatiques reste considérable. En effet, les infections sont les premières causes de mortalité secondaire en traumatologie lourde. La survenue d'une infection chez un patient traumatisé grave élève la mortalité prédite de 7 à 21%, augmente la durée d'hospitalisation en réanimation de 5 à 21 jours en moyenne et prolonge la durée d'hospitalisation de 7 à 34 jours (Rincon-Ferrari et al. 2004 ; Papia et al. 1999). A titre d'exemple, le surcoût global lié aux pneumopathies acquises sous ventilation mécanique (PAVM) est évalué, en France, à 800 millions d'euros par an. Enfin, selon un rapport de l'OMS paru en 2005, le coût du traitement de 5 infections nosocomiales de gravité moyenne équivaut à l'ensemble du budget annuel prévu pour l'achat des produits d'hygiène des mains d'un service d'hospitalisation.

Cela traduit, d'une part, l'inefficacité des moyens actuels de prévention des infections nosocomiales pour cette population particulière de patients et, d'autre part, l'importance médico-économique de ce problème de santé publique (SFAR 2005).

Les altérations de présentation de l'antigène sont une cause majeure du sepsis consécutif à un traumatisme ou un polytraumatisme sévère. Les capacités de présentation de l'antigène ont été étudiées dans le cadre du stress aigu, notamment septique ou traumatique. Ces études ont essentiellement concerné l'expression de HLA-DR sur des monocytes sanguins. Chez les patients souffrant d'un traumatisme grave, il a été démontré que l'expression membranaire monocytaire de HLA-DR était significativement inférieure à celle observée chez des volontaires sains (Venet et al. 2007). Ditschkowski et al. (1999) ont étudié l'expression membranaire monocytaire de HLA-DR chez 66 polytraumatisés sévères pendant les 14 premiers jours d'hospitalisation. Les vingt patients qui ont présenté un sepsis au cours de leur évolution présentaient dès J0 et jusqu'à J14 une diminution importante de l'expression de HLA-DR monocytaire par rapport aux 46 polytraumatisés n'ayant pas présenté d'infections au cours de leur séjour. Une diminution de l'expression d'HLA-DR a également été rapportée chez des patients chirurgicaux et, là encore, cette constatation s'accompagnait d'une augmentation des complications septiques et d'une surmortalité chez ces patients (Ditschkowski et al. 1999). Il apparaît, au vu de ces données que, très rapidement après la survenue du traumatisme, s'instaure une phase de désactivation du système immunitaire marquée par une diminution de la capacité à présenter l'antigène par les monocytes (diminution d'expression de HLA-DR). C'est précisément cette atteinte bien spécifique du système immunitaire des patients, consécutivement à un traumatisme ou polytraumatisme sévère, qui favorise la survenue d'infections nosocomiales et est corrélée à une surmortalité préoccupante.

A l'heure actuelle, la physiopathologie du traumatisme sévère et de ses complications secondaires reste basée sur des considérations cliniques exclusives. Or, les Inventeurs sont les premiers à proposer une approche originale tenant compte de l'état physiologique et pathologique, en particulier immunologique, des patients souffrant de traumatismes sévères. Comme cela est décrit en détail ci-après, les Inventeurs ont développé le premier modèle animal reproduisant l'IS post-traumatique observée chez l'homme et combinée à une pneumopathie, afin de mimer la pathologie humaine de la manière la plus réaliste possible. Grâce à leurs travaux, réalisés notamment à l'aide de ce modèle animal, les Inventeurs sont parvenus à expliquer l'incidence des complications septiques de l'IS post-traumatique chez les patients hospitalisés pour traumatismes sévères, et proposent pour la première fois des moyens adaptés et efficaces pour prévenir ces complications.

Depuis une dizaine d'années et consécutivement à la découverte des TLRs, l'immunité innée est devenue un sujet d'étude particulièrement répandu. L'immunité innée permet une défense anti-infectieuse spécifique très efficace, le plus souvent suffisante pour éradiquer un foyer infectieux. L'étude de l'immunité innée a permis de découvrir qu'elle reconnaît très spécifiquement des motifs moléculaires conservés chez de nombreux pathogènes, les PAMP (pour « pathogen-associated molecular patterns »), essentiellement par le biais des TLRs appartenant à la famille des « Pattern Recognition Receptors » (PRRs). Les PAMPs sont d'origine très diverse (bactérie, virus, parasite, etc.) et de nature variée (protéine, sucre, acide nucléique, etc.) En termes de structure, les TLRs sont des protéines transmembranaires de type I comportant un domaine extracellulaire récepteur du signal de danger et composé de nombreux motifs du type « répétitions riches en leucine » ou LRRs (pour « leucin-rich repeats »), un domaine transmembranaire et un domaine intracellulaire contenant un domaine permettant la transduction du signal d'activation (appelé « death domain »). Les TLRs sont largement exprimés par de nombreux types de cellules, par exemple des cellules du sang, de la rate, des poumons, des muscles, des intestins, etc. Les TLRs représentent, chez les mammifères notamment, des protéines clés permettant de détecter une infection et de déclencher une réponse immunitaire appropriée. La plupart des espèces de mammifères compte entre 10 et 15 types de TLRs. En particulier, 13 TLRs (TLR 1 à TLR 13) ont été identifiés chez la souris, et 10 chez l'homme. Chaque TLR est capable de reconnaître spécifiquement certains PAMPs. Par exemple, le récepteur TLR 4 a pour ligands naturels le LPS de la paroi bactérienne et des glycoprotéines d'origine virale. Le récepteur TLR 9, quant à lui, reconnaît les îlots CpG non-méthylés de l'ADN bactérien et viral.

Ainsi, les TLRs et leurs agonistes apparaissent comme des cibles majeures de recherche pour le développement de thérapeutiques efficaces dans le domaine de la lutte contre les infections.

Parmi les agonistes connus des TLRs, on peut notamment citer le MPLA et les CpG. Le MPLA ou lipide A monophosphoryle est un dérivé non toxique du lipopolysacchardie (LPS) issu d'une souche de *Salmonella minnesota.* Il agit essentiellement via le TLR 4. En termes de structure, le MPLA et le MPLA 3-O désacylaté (ou 3D-MPLA) possèdent un squelette glucidique sur lequel sont fixés des acides gras à longue chaîne. Ce sont des molécules très hydrophobes. Le MPLA et le 3D-MPLA sont classiquement utilisés en tant qu'adjuvants d'immunité associés à des préparations antigéniques dans des vaccins de prévention contre les infections (par exemple, contre le virus de la grippe, influenza) ou des vaccins thérapeutiques pour le traitement de cancers et d'infections chroniques (voir, par exemple, le brevet européen EP 0 971 739).

Les motifs CpG sont de courtes molécules d'ADN simple brin contenant une cytosine « C » suivie d'une guanine « G ». La lettre « p » dans l'acronyme « CpG » désigne le squelette phosphodiester de l'ADN. Les oligodésoxynucléotides CpG (ou CpG ODN) synthétiques se distinguent des CpG ODN microbiens par leur squelette partiellement ou complètement modifié en phosphorothioate « PS » (au lieu de phosphodiester) et par une queue polyG à leur(s) extrémité(s) 5' et/ou 3'. La modification PS empêche la dégradation des CpG ODN par les nucléases et la queue polyG améliore la prise en charge par les cellules. Les CpG naturels sont particulièrement abondants dans les génomes microbiens et beaucoup plus rares chez les vertébrés. Ils sont exclusivement reconnus par le TLR 9. Les CpG non-méthylés sont utilisés dans des compositions vaccinales en tant qu'adjuvants (voir, par exemple, le brevet européen EP 0 772 619).

Des compositions immunostimulantes comprenant un ou plusieurs ligands des TLRs ont déjà été proposées à des fins thérapeutiques et/ou prophylactiques. Ainsi, la demande internationale WO 2009/088401 propose d'améliorer la réponse immunitaire d'un sujet à l'aide de telles compositions. Des compositions incluant des CpG et/ou du MPLA sont ainsi décrites pour une administration à un sujet présentant un risque supérieur à la normale de développer, dans un avenir indéfini, une infection, un cancer ou une allergie. Les applications anti-infectieuses envisagées dans cette demande sont purement vaccinales : il s'agit en effet d'immuniser un sujet afin de le protéger contre un agent infectieux auquel il pourrait être exposé au cours de sa vie. Or, typiquement, dans le domaine de la vaccinothérapie, nul ne sait : (i) si le sujet vacciné sera effectivement exposé au cours de sa vie à l'agent infectieux contre lequel il est censé être protégé ; (ii) en admettant que le sujet soit exposé à l'agent infectieux, quand aura lieu cette exposition ; (ii) si le sujet vacciné est efficacement protégé pour ne pas déclarer d'infection en cas d'exposition à l'agent infectieux ; et (iii) en supposant qu'il y ait protection associée au vaccin, quelle est la durée réelle de cette protection vis-à-vis d'une exposition à l'agent infectieux. De plus, l'enseignement contenu dans la demande WO 2009/088401 est insuffisant à démontrer l'efficacité des compositions vaccinales décrites.

Au contraire, dans le cadre de la présente invention, d'une part, les compositions proposées ne sont pas vaccinales au sens classique du terme et, d'autre part, la population à traiter est bien spécifique. Les infections à prévenir dans le cadre de la présente invention sont les complications septiques consécutives à l'immunodépression systémique post-traumatique chez les patients hospitalisés pour traumatismes sévères. Ces complications ne sont susceptibles de survenir que dans un délai bien précis à compter du jour du ou des traumatismes. De plus, la présente invention vise une catégorie bien particulière de patients, dont l'état physiologique et pathologique, en particulier immunologique, diffère de celui de la population cible d'une vaccination standard. Comme indiqué précédemment, les patients visés ici sont malades : ils sont atteints d'un ou plusieurs traumatismes sévères, alors que la population cible d'une campagne de vaccination classique est généralement saine. L'état pathologique des patients ici visés n'est à ce point pas anodin qu'il justifie une prise en charge et une surveillance par des équipes médicales compétentes, et qu'il nécessite une hospitalisation d'urgence, relativement durable, typiquement dans un ou plusieurs services hospitaliers spécialisés (réanimation, traumatologie lourde...) Comme indiqué plus haut, ces patients présentent une IS post-traumatique spécifique, sévère et prolongée qui est une conséquence directe du ou des traumatismes sévères qu'ils ont subis. Les caractéristiques propres à l'IS post-traumatique et aux complications septiques qui y sont associées sont détaillées dans la description qui suit.

Ainsi, la présente invention a pour objet une composition pharmaceutique comprenant au moins un agoniste du récepteur TLR 4, pour son utilisation dans le traitement prophylactique (ou la prévention) des complications septiques de l'immunodépression systémique post-traumatique chez un patient hospitalisé atteint d'un ou plusieurs traumatismes sévères, ledit agoniste étant choisi parmi le lipide A monophosphoryle (MPLA) et le lipide A monophosphoryle 3-O désacylaté (3D-MPLA).

Plus généralement, la présente description décrit une composition pharmaceutique comprenant au moins un agoniste d'au moins un TLR choisi parmi les TLR 4 et 9, pour son utilisation dans le traitement prophylactique (ou la prévention) des complications septiques de l'immunodépression systémique post-traumatique chez un patient hospitalisé atteint d'un ou plusieurs traumatismes sévères.

L'invention vise une population bien particulière de patients. Les patients considérés sont atteints d'un ou plusieurs traumatismes sévères. Pour simplifier, on pourra parler de « patients traumatisés graves » ou « patients traumatisés sévères ». De préférence, les patients en question présentent au moins deux lésions traumatiques parmi lesquelles au moins une lésion engage le pronostic vital (c'est-à-dire qu'elle peut tuer à court terme). De manière encore préférée, les patients traumatisés sévères présentent un score de gravité des lésions (« Injury Severity Score » ou ISS) d'au moins 16. Pour les traumatismes très sévères, l'ISS est d'au moins 25. Le calcul du score ISS prend en compte l'atteinte de plusieurs régions de l'organisme du patient (tête et cou ; face ; thorax ; abdomen et pelvis ; bassins et membres ; peau et tissus sous cutanés). L'atteinte de chaque région est cotée de 1 à 6 en fonction de sa gravité (1 : atteinte mineure, 6 : atteinte critique). L'ISS est ensuite calculé par la mise au carré du score des trois régions les plus atteintes (par exemple, si les côtes de chaque région sont les suivantes : tête 4, abdomen 3, thorax 2, autres régions 1, alors l'ISS vaudra $4^2+3^2+2^2 = 16+9+4 = 29$ (Baker et al. 1974). Alternativement ou additionnellement, les patients traumatisés considérés dans le cadre de l'invention présentent un traumatisme crânien sévère défini par un score de Glasgow (CGS) inférieur à 8. La détermination du CGS est une méthode qui permet d'apprécier la profondeur d'un coma par l'étude de la variabilité de 3 critères cliniques très précis qui sont : 1) l'ouverture des yeux (coté de 1 : absente à 4 : ouverture spontanée des yeux), 2) les capacités de motilité (faculté de se mouvoir), ou si l'on préfère la meilleure réponse motrice (cotée de 1 : absente à 6 : mouvements adaptés), et 3) la réponse aux questions posées (réponses verbales, cotée de 1 : absente à 5 : réponse orientée). Le CGS est la somme des résultats obtenus aux trois critères cliniques cités ci-dessus. Il est donc au minimum de 3 et au maximum de 15 (Teasdale et al. 1974).

Comme expliqué supra, la population particulière de patients visée dans l'invention se distingue par une IS post-traumatique bien spécifique.

Cette IS post-traumatique se caractérise de préférence par :

> a) une baisse du niveau de production *ex vivo* de cytokines pro-inflammatoires induites par des leucocytes sanguins après stimulation par du LPS de bacilles gram négatif, par exemple *Escherichia coli,* par rapport au niveau de production observé pour un individu sain ; et/ou
> b) une baisse du niveau d'expression de HLA-DR sur les cellules présentatrices des antigènes des patients, par rapport au niveau d'expression observé pour un individu sain.

Ces caractéristiques de l'IS post-traumatique ont été confirmées par la littérature (Keel et al. 1996; Spolarics et al. 2003; Cheadle et al. 1991). La diminution observée du niveau de production de cytokines (exprimé par exemple en picogrammes/ml) s'entend versus volontaires sains dont le niveau de production de cytokines représente la valeur 100%. Les cytokines sont typiquement mesurées dans des cultures de sang total stimulées par du LPS (*d'Escherichia coli* en général). L'expression de HLA-DR s'exprime versus volontaires sains, soit en nombre de molécules d'HLA-DR exprimées à la surface des cellules (MFI pour « mean fluorescence intensity »), soit en pourcentage, la valeur 100% représentant le niveau d'expression de HLA-DR chez des volontaires sains. De manière particulière, si l'on raisonne en pourcentage, la baisse en a) et/ou b) ci-dessus est d'au moins 20% environ. Elle est de préférence d'au moins 25% environ, de préférence encore d'au moins 30, 35, 40, 45% environ, et de manière plus préférentielle d'au moins 50, 55, 60% environ, voire plus.

De préférence encore, l'IS post-traumatique observée chez les patients traumatisés sévères est telle que le niveau d'expression de HLA-DR sur les monocytes desdits patients dans les 24 heures suivant le ou les traumatismes (de J0 à J1) est diminué par rapport au niveau d'expression observé pour un individu sain. Cette diminution initiale peut d'ailleurs permettre, en pratique, de prédire le risque de complications septiques (ou infections secondaires). Ainsi, un bas niveau d'expression de HLA-DR sur les monocytes le premier jour suivant le ou les traumatismes sévères (J1), par exemple une diminution de 50%, est prédictif d'un risque élevé pour le patient de contracter une infection secondaire. L'expression de HLA-DR s'exprime versus volontaires sains, soit en nombre de molécules d'HLA-DR exprimées à la surface des cellules (MFI pour « mean fluorescence intensity »), soit en pourcentage, la valeur 100% représentant le niveau d'expression de HLA-DR chez des volontaires sains. En particulier, si l'on raisonne en pourcentage, la diminution du niveau d'expression de HLA-DR est d'au moins 20% environ, de préférence d'au moins 25% environ, de préférence encore d'au moins 30, 35, 40, 45% environ, et de manière plus préférentielle d'au moins 50, 55, 60% environ, voire plus.

A la lumière des paragraphes précédents, un homme du métier comprendra aisément que l'état pathologique de la population particulière de patients visée par la présente invention peut être défini de manière nécessaire et suffisante par les caractéristiques sus-mentionnées. Cet état pathologique et les risques de complications septiques qui y sont associés interviennent dans une échelle de temps limitée et tout à fait spécifique, suite au(x) traumatisme(s) ayant conduit lesdits patients dans un service hospitalier de réanimation. En particulier, l'immunodépression systémique post-traumatique dont il est question dans le cadre de la présente invention met en jeu des mécanismes anti-inflammatoires et se distingue à ce titre des états pathologiques impliquant des réactions pro-inflammatoires de type SIRS, notamment dans le cadre du sepsis.

Les complications septiques (ou infections secondaires) que la composition pharmaceutique objet de la présente invention permet de prévenir sont plus particulièrement des infections nosocomiales, notamment bactériennes. En particulier, les infections bactériennes en cause sont choisies parmi les pneumopathies, telles que les PAVMs, les infections urinaires, les infections sur cathéters veineux centraux, les infections bactériennes cérébroméningées telles méningites empyèmes et abcès cérébraux. Plus particulièrement encore, les pneumopathies sont dues à des bactéries pathogènes choisies parmi les staphylocoques, de préférence *Staphylococcus aureus,* de préférence encore *Staphylococcus aureus* sensible à la méticilline, *Haemophilus sp.*, de préférence *H. influenzae,* les pneumocoques, les entérobactéries, *Pseudomonas* sp., en particulier *P. aeruginosa.* Les bactéries à l'origine des pneumopathies peuvent également appartenir à d'autres genres ou espèces bactériens (par exemple, des bacilles gram négatif et des coques gram positif). De plus, elles peuvent être résistantes aux antibiotiques. S'agissant des infections autres que les pneumopathies, les bactéries responsables peuvent être, par exemple, des bacilles gram négatif (e.g., *E. coli, Proteus mirabilis, Pseudomonas aeruginosa*) et des coques gram positif (e.g., *S. aureus*) pour les infections urinaires ; des bacilles gram négatif (e.g., *E. coli, P. mirabilis, P. aeruginosa*), des coques gram positif (e.g., *S. aureus,* des staphylocoques coagulase négatif) et des levures telles que *Candida* sp. pour les infections sur cathéters centraux ; des bacilles gram négatif (e.g., *E. coli, P. mirabilis, P. aeruginosa*) et des coques gram positif (e.g., *S. aureus,* des staphylocoques coagulase négatif, *Streptococcus pneumoniae*) pour les infections bactériennes cérébroméningées.

On comprendra que, dans la composition pharmaceutique selon la présente invention, le ou les agonistes de TLRs est(sont) présent(s) en quantité efficace (ou quantité pharmaceutiquement efficace). Cela signifie que la quantité d'agonistes dans la composition est telle qu'une fois administrée, celle-ci est capable de prévenir les complications septiques de l'immunodépression systémique post-traumatique chez un patient hospitalisé atteint d'un ou plusieurs traumatismes sévères.

La composition pharmaceutique décrite dans la présente description peut comprendre au moins un agoniste du TLR 4 et au moins un agoniste du TLR 9.

Le terme « agoniste » désigne ici n'importe quelle molécule ou combinaison de molécules qui stimule un récepteur. Par exemple, un agoniste d'un TLR peut être un ligand d'un TLR, un analogue ou un dérivé d'un tel ligand. Par « molécule ou combinaison de molécules stimulant un récepteur TLR », on désigne ici des molécules qui déclenchent, via le dit TLR (directement ou indirectement), une cascade de signalisation intracellulaire spécifique. Cette cascade de signalisation fait intervenir de nombreuses protéines adaptatrices telles que MyD88, MAL, TRIF et TRAM. Les agonistes de TLR4 et TLR9 activent tous deux la voie de signalisation MyD88. Toutefois, les agonistes de TLR4 font également intervenir les protéines MAL, TRIF et TRAM, ce qui n'est pas le cas des agonistes de TLR9.

Le recrutement de ces protéines adaptatrices conduit entre autres à l'activation de macrophages et de cellules dendritiques (DC), ainsi qu'à la production de cytokines pro-inflammatoires, chimiokines et immunoglobulines. Les agonistes de TLR4 activent plus particulièrement les macrophages et cellules dendritiques myéloïdes tandis que les agonistes de TLR9 activent les macrophages, les cellules dendritiques plasmacytoïdes (pDC) ainsi que les lymphocytes B.

Un ligand d'un TLR peut être de nature chimique (par exemple, le MPLA) ou biologique (par exemple, un composé étranger reconnu par le système immunitaire comme du non-soi, tel que les acides nucléiques de bactéries, champignons ou virus, et plus particulièrement l'acide désoxyribonucléique de *Candida albicans* ou les CpG). Des analogues ou dérivés de ligands de TLRs sont notamment des ligands structurellement modifiés (e.g., mutés si ce sont des molécules biologiques ou modifiés dans leur structure si ce sont des molécules chimiques), des peptides mimétiques, et plus généralement n'importe quelle molécule ou combinaison de molécules capable de remplir la fonction biologique du ligand, qui est de stimuler le récepteur.

L'agoniste du TLR 4 contenu dans la composition selon l'invention est le lipide A monophosphoryle (MPLA) ou le lipide A monophosphoryle 3-O désacylaté (3D-MPLA). On utilisera plus particulièrement le MPLA.

De préférence, l'agoniste du TLR 9 contenu dans une composition décrite dans la présente description est un oligodésoxynucléotide CpG (CpG ODN). On préférera utiliser un CpG ODN synthétique d'au moins 8 nucléotides contenant au moins un dinucléotide CpG non méthylé. De préférence, on utilisera un CpG ODN contenant au plus 100 nucléotides. De préférence encore, le CpG ODN utilisé dans la composition décrite dans la présente description contiendra de 8 à 40 nucléotides. L'homme du métier choisira le ou les CpG ODN appropriés parmi les phosphodiesters ou les phosphodiesters comprenant une ou plusieurs modifications en phosphorothioate (phosphodiester / phosphorothioate), ou encore les phosphorothioates. On préférera utiliser des CpG ODN phosphodiesters / phosphorothioates, ou phosphorothioates. Avantageusement, on utilisera des CpG ODNs dépourvus de séquences palindromiques de 4 à 8 nucléotides environ. On pourra choisir d'utiliser un CpG ODN autre que le CpG 7909 (ou PF-3512676) décrit dans Léonard et al. (2007).

De préférence, la composition pharmaceutique selon l'invention ou une composition décrite dans la présente description comprend en outre un ou plusieurs excipients ou additifs ou supports pharmaceutiquement acceptables. Par exemple, la composition selon la présente invention ou une composition décrite dans la présente description peut comprendre un ou plusieurs agents choisis parmi les diluants, agents anti-mousse, stabilisants, colorants, stabilisants, conservateurs, etc. On préférera utiliser des agents inertes, ce qui signifie que, dans les compositions objets de l'invention et celles décrites dans la présente description, les seuls agents actifs du point de vue du traitement prophylactique ici recherché, seront le ou les agonistes des TLRs. Néanmoins, la composition pharmaceutique objet de la présente invention ou une composition décrite dans la présente description pourra comprendre un ou plusieurs antigènes, en sus desdits agonistes des TLRs. Le ou les antigènes, ainsi associés à l' (aux) agoniste(s) du(es) TLR(s), pourront permettre d'obtenir une immunostimulation puissante et spécifique du ou des antigènes ajouté(s) dans la composition pharmaceutique selon l'invention ou dans une composition décrite dans la présente description. Les antigène(s) éventuellement mis en oeuvre sont principalement des antigènes bactériens, en particulier des antigènes de bactéries pathogènes telles que les staphylocoques, de préférence *Staphylococcus aureus*, de préférence encore *S. aureus* sensible à la méticilline, *Haemophilus* sp., de préférence *H. influenzae*, les pneumocoques, les entérobactéries, *Pseudomonas* sp., en particulier *P. aeruginosa.* Les antigènes pourront provenir d'autres genres ou espèces bactériens en fonction des infections que l'on souhaite prévenir (par exemple, des bacilles gram négatif tels que *E. coli*, *Proteus mirabilis*, etc., des coques gram positif tels que *Streptococcus pneumoniae*, et des levures telles que *Candida* sp.).

Avantageusement, la composition selon la présente invention ou une composition décrite dans la présente description est administrée aux patients, par exemple par injection, à partir de 12h environ, de préférence à partir de 24h environ, à compter de leur admission à l'hôpital, en particulier dans un service hospitalier de réanimation. En particulier, la composition est administrée après stabilisation de l'état clinique des patients. Généralement, un patient est stabilisé lorsque, selon le ou les types de lésions qu'il présente, les saignements sont contrôlés et/ou les lésions menaçant le pronostic vital à court terme ont été traitées chirurgicalement en urgence. En pratique, un patient est dit « stabilisé » si ses fonctions vitales (circulatoire et respiratoire essentiellement) sont stables.

La composition selon la présente invention ou une composition décrite dans la présente description est de préférence administrée, une ou plusieurs fois si nécessaire, dans une période d'au plus 1 mois environ, de préférence d'au plus 28 jours, à compter de l'admission des patients à l'hôpital, en particulier dans un service hospitalier de réanimation. En d'autres termes, les complications septiques que l'on veut prévenir sont susceptibles de survenir (ou d'être contractées) pendant au plus 1 mois environ, de préférence au plus 28 jours, à compter de l'admission des patients à l'hôpital. Par exemple, on a pu constater que des complications septiques survenaient chez 40 à 50% des patients traumatisés sévères entre J0 et J10, et chez 10 à 20% des patients en plus entre J10 et J28, soit 50 à 70 % des patients au total avaient contracté une infection secondaire (Osborn et al., 2004).

En particulier, la composition pharmaceutique objet de la présente invention ou une composition décrite dans la présente description pourra être administrée une ou plusieurs fois pendant la période d'intubation des patients.

Ainsi, la présente description se rapporte également à une méthode de traitement prophylactique des complications septiques de l'immunodépression post-traumatique chez un patient hospitalisé atteint d'un ou plusieurs traumatismes sévères, dans laquelle on administre audit patient une composition pharmaceutique telle que décrite ci-dessus.

La présente description a également pour objet l'utilisation d'au moins un agoniste d'au moins un TLR choisi parmi les TLR 4 et 9, pour la préparation d'un médicament destiné au traitement prophylactique des complications septiques de l'immunodépression systémique post-traumatique chez un patient hospitalisé atteint d'un ou plusieurs traumatismes sévères.

De préférence, ledit médicament est une composition pharmaceutique conforme à la description qui précède.

La présente invention est illustrée par les figures suivantes :

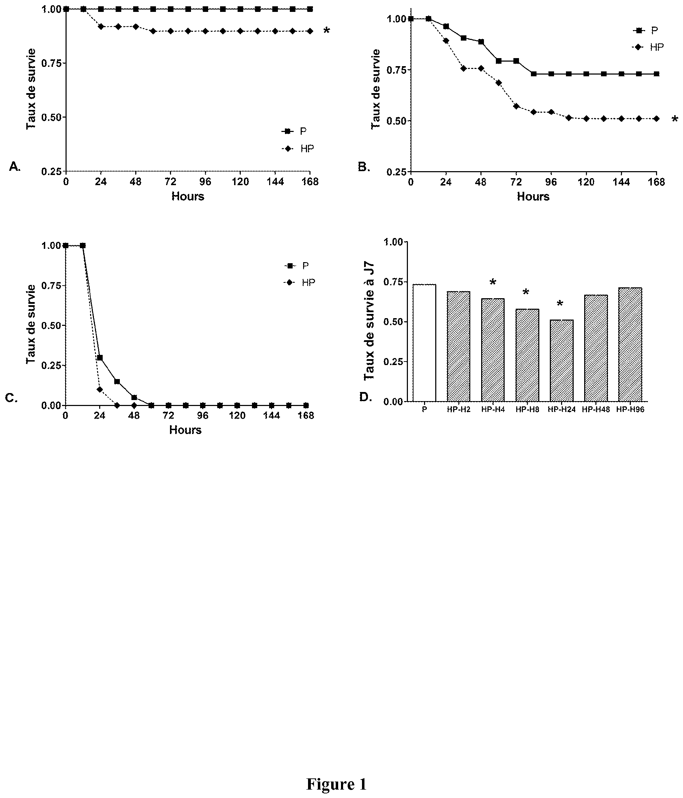

- Figure 1: Etude pilote des effets de l'inoculum et du délai entre choc hémorragique et pneumopathie à *Staphyloccocus aureus* methicilline sensible (ou « SASM ») sur la mortalité des souris.

 Deux groupes de souris ont été étudiées : groupe HP (choc hémorragique suivi d'une pneumopathie à SASM) et groupe P (pneumopathie à SASM seule). Les données sont exprimées en pourcentage et sont représentatives de trois expériences indépendantes, chacune ayant la même significativité statistique. *A.B.C.* Effet de l'inoculum sur la mortalité. Vingt quatre heures après l'hémorragie, une pneumopathie a été réalisée avec $7 \times 10^5$ UFC *(A.)* ou $7 \times 10^6$ UFC *(B.)* ou $7 \times 10^7$ UFC *(C.)* de SASM (groupe HP) et comparée à un groupe de souris avec une pneumopathie seule (groupe P). Le taux de survie de chaque groupe a été contrôlé deux fois par jour pendant 7 jours (168 heures). Chaque groupe comprenait 15 souris. (* $p \leq 0.05$ versus group P). *D.* Effet du délai sur la mortalité. La mortalité a été évaluée sur des souris soumises à une pneumopathie à SASM ($7.10^6$ CFU) réalisée 2, 4, 8, 24, 48 et 96 après l'hémorragie (HP-H2, -H4, -H8, -H24, -H48 et -H96, respectivement) et comparée à un groupe de souris soumises à une pneumopathie à SASM seule (groupe P). Chaque groupe comprenait 15 animaux. (* significativement différent du groupe P après correction de Bonferroni).

- Figure 2 : Représentation schématique des 3 groupes de l'étude principale. C, groupe contrôle ; P, groupe Pneumonie ; HP, groupe Hémorragie Pneumonie.

- Figure 3 : Le choc hémorragique aggrave la perte de poids et les conséquences biologiques d'une pneumopathie à SASM.

 Les données sont exprimées en médiane +/- SEM et sont représentatives de trois expériences indépendantes, chacune ayant la même signification statistique.

 **A.** Les souris ont été pesées tous les jours pendant 5 jours (120 heures). Chaque groupe comprenait 10 souris. (& $p \leq 0.001$ versus groupe C, * $p \leq 0.01$ versus groupe P). **B.** Les échantillons sanguins ont été prélevés dans l'oreillette droite 24 heures après le début de la pneumopathie. Chaque groupe comprenait 5 animaux. (* $p \leq 0.05$ versus groupe C, & $p \leq 0.05$ versus group P).

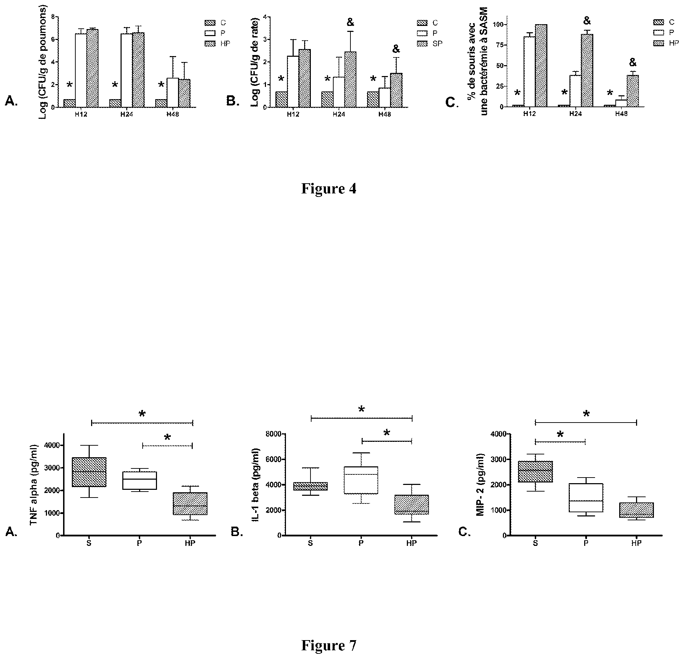

- Figure 4: L'IS post hémorragique induit des bactériémies au cours d'une pneumopathie à SASM.

 Les animaux ont été sacrifiés 12, 24 et 48 heures après le début de la pneumopathie. Les comptes de colonies de SASM dans les poumons et dans la rate ont été.réalisés après culture sur milieu sélectif. Les données présentées incluent 15 souris par groupe regroupant 3 expériences indépendantes, chacune ayant la même signification sta-tistique. (* $p \leq 0.05$ versus tous les autres; & $p \leq 0.05$ versus le groupe P au temps indiqué).

 Evolution locale (A) et systémique (B.C.) de l'inoculum bactérien. Les données représentées sont des médianes $\pm$ SEM **(A.,B.)** ou des pourcentages $\pm$ SEM **(C.).** Le seuil de détection de la méthode était de 0,7 UFC par gramme de tissu.

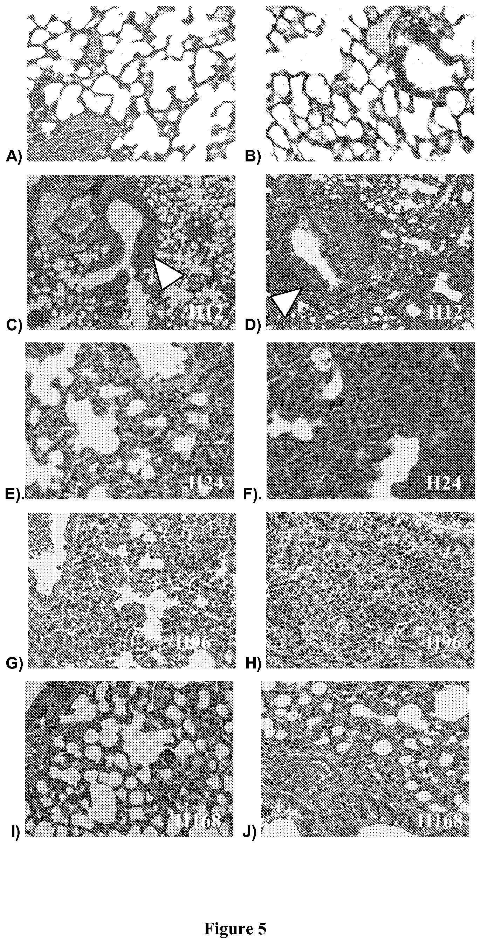

- Figure 5: L'IS post hémorragique accentue les lésions histologiques d'une pneumopathie à SASM.

 Quatre groupes de souris ont été étudiés: Naïve ; Contrôle (groupe C); pneumopathie à SASM seule (groupe P) et hémorragie suivie d'une pneumopathie à SASM (groupe HP). Les tissus fixés dans le formol ont été coupés puis colorés par hematoxyline et éosine avant analyse microscopique *(grossissement x 20).*

 Aspect histologique représentatif des poumons de **(A.)** souris naïve ayant un poumon sain ; **(B)** souris du groupe contrôle à la 24ème heure. Le parenchyme pulmonaire est ensuite présenté par une série d'images obtenues sur des animaux à différents temps de la pneumopathie: 12, 24, 96 et 168 heures **(C.E.G.I.)** pour le groupe P ; et **(D.F.H.J)** pour le groupe HP. Les agrégats de cellules immunes apparaissaient dès la 12ème heure de l'infection (flèches) et étaient plus nombreux dans le groupe HP que dans le groupe P à tous les délais.

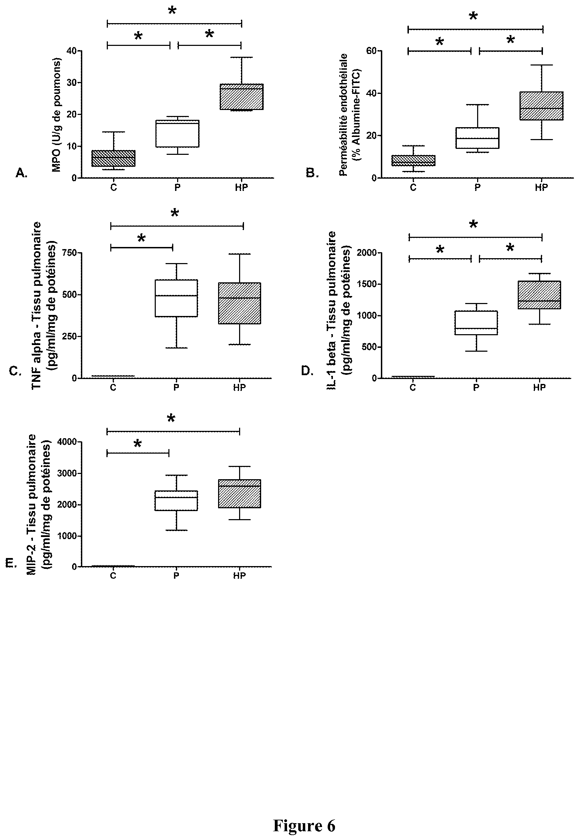

- Figure 6: L'IS post hémorragique aggrave les lésions inflammatoires d'une pneumopathie à SASM

 Chaque groupe de souris comprenait 5 animaux. Les données sont exprimées en médiane $\pm$ 25-75ème percentiles et sont représentatives de trois expériences indépendantes, chacune ayant la même signification statistique. (* $p \leq 0.05$).

 **A.** Accumulation de polynucléaires neutrophiles dans les poumons. L'activité myélopéroxydasique (MPO) a été mesurée dans les deux poumons.

*B.* Lésions pulmonaires endothéliales. La perméabilité endothéliale a été déterminée par la mesure du pourcentage d'albumine FITC passant à travers les capillaires pulmonaires.

*C.D.E.* Concentrations pulmonaires des cytokines. Les concentrations en TNF alpha **(C.),** IL-1 beta *(D.)* et MIP-2 *(E.)* ont été mesurées dans les broyats de poumons.

- Figure 7: L'IS post hémorragique majore l'hypo-réactivité sanguine au LPS durant une pneumopathie à SASM. Un échantillon de sang total a été cultivé 24 heures avec du LPS issu d'*E.coli O111B4* et les cytokines ont été mesurées dans les surnageants des cultures cellulaires. Chaque groupe comprenait 5 animaux. Les données ont été exprimées en médiane $\pm$ 25-75$^{ème}$ percentiles et sont représentatives de trois expériences indépendantes, chacune ayant la même signification statistique. (* p $\leq$ 0.05).

Les concentrations en TNF alpha **(A.),** IL-1 beta **(B.)** et MIP-2 **(C.)** dans les cultures cellulaires non stimulées par LPS étaient toutes sous le seuil de détection du dosage.

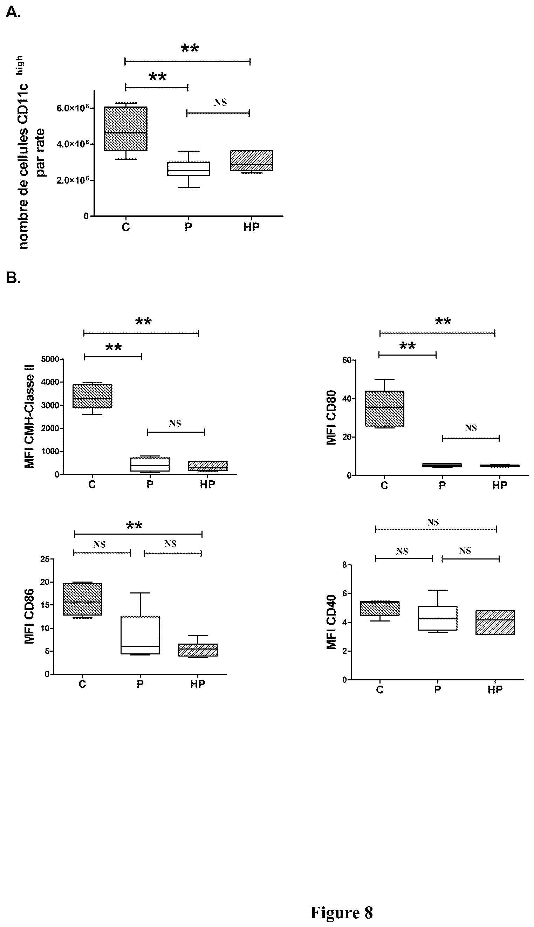

- Figure 8: Nombre et état de maturation des cellules spléniques CD11C$^{high}$ au cours d'une pneumopathie à SASM précédée ou non d'un choc hémorragique. Chaque groupe de souris comprenait 6 animaux. Les données ont été exprimées en médiane $\pm$ 25-75$^{ème}$ percentiles et sont représentatives de deux expériences indépendantes, chacune ayant la même signification statistique. (** p $\leq$ 0.01).

*A*. Numération des cellules CD11c$^{high}$ dans la rate. Dans chaque groupe, la suspension cellulaire obtenue par digestion enzymatique des rates a donné lieu à une numération puis le pourcentage de cellules CD11c$^{high}$ a été déterminé par FACS. Le nombre absolu de cellules CD11c$^{high}$ a alors été calculé.

*B.* Expression des marqueurs de maturation des CD11c$^{high}$. Les marqueurs membranaires suivants ont été analysés par FACS: CMH-Classe II, CD40, CD80, CD86.

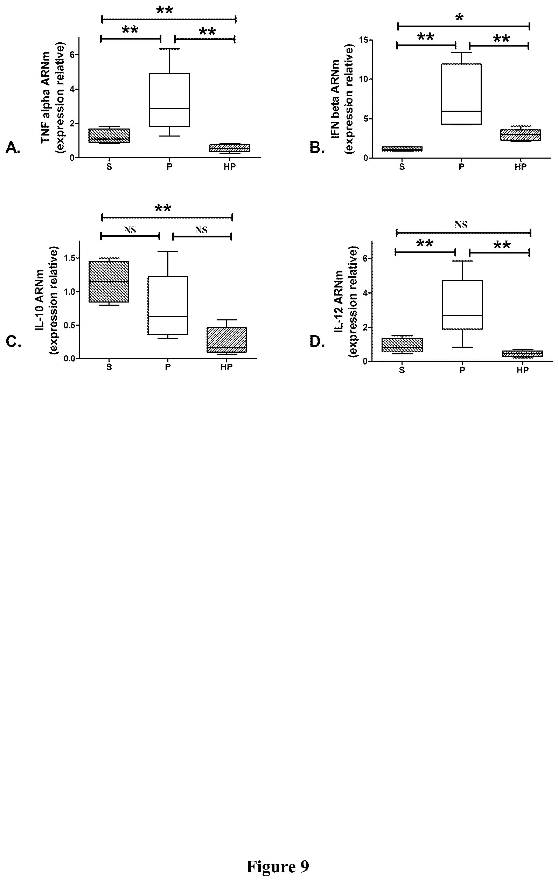

- Figure 9: L'IS post hémorragique diminue la transcription: (A.) d'une cytokines dépendante de Nuclear Factor-kB (NF-kB), (B.) d'une cytokine dépendant de l'Interferon Regulatory Factor (IRF-7), (C.) de l'Interleukine (IL-10) et (D.) de l'IL-12 dans les CD spléniques au décours d'une pneumopathie à SASM.

Chaque groupe de souris comprenait 6 animaux. Les données ont été exprimées en médiane $\pm$ 25-75$^{ème}$ percentiles et sont représentatives de deux expériences indépendantes, chacune ayant la même signification statistique. (* p $\leq$ 0.05 ; ** p $\leq$ 0.01).

**A. B. C. D.** Analyse en RT-PCR quantitative de **(A.)** Tumor Necrosis Factor (TNF alpha) ARNm; **(B.)** Interferon (IFN beta) ARNm; **(C.)** IL-10 ARNm et **(D.)** IL-12 ARNm dans les CD spléniques. Les ARNm ont été extraits des cellules CD11 c+ sélectionnés positivement dans les suspensions cellulaires spléniques.

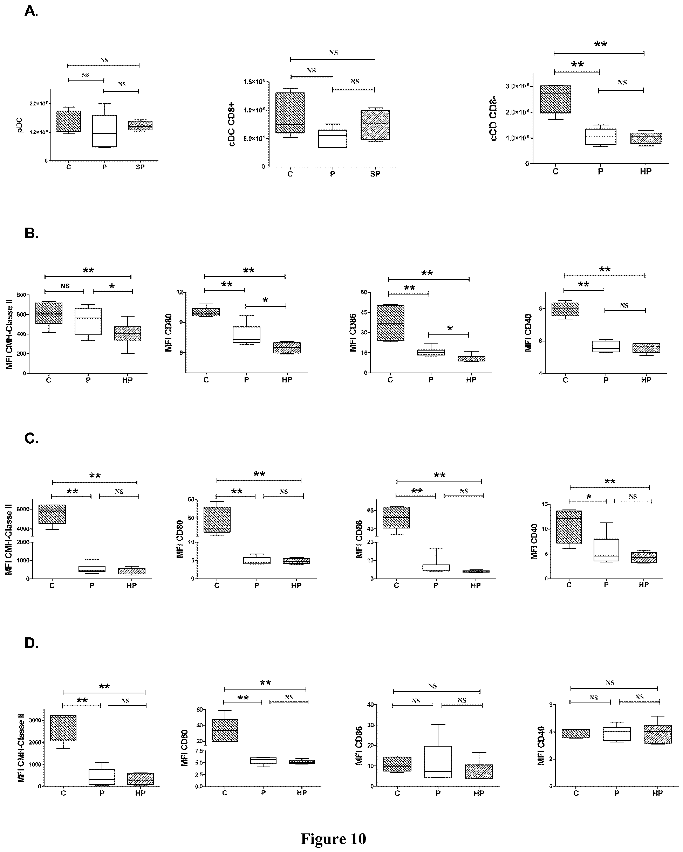

- Figure 10: Altérations des phénotypes des sous-populations de cellules dendritiques liées à l'IS post hémorragique lors d'une pneumopathie à SASM. Chaque groupe de souris comprenait 6 animaux. Les données ont été exprimées en médiane $\pm$ 25-75$^{ème}$ percentiles et sont représentatives de deux expériences indépendantes, chacune ayant la même signification statistique. (* p $\leq$ 0.05 ; ** p $\leq$ 0.01).

Nombre de cellules de chaque sous population de CD spléniques **(A.),** Capacités de présentation de l'antigène et état de maturation des pCD **(B.) ;** des CD conventionnelles cCD CD8+ (cCD CD8+) **(C.)** et cCD CD8- **(D.)** dans la rate suivant une pneumopathie à SASM.

Numération de sous population splénique de CD. Les cellules ont été obtenues après digestion enzymatique des rates. Un compte sur cellule de Malassez puis la détermination du pourcentage de chaque type cellulaire ont été réalisés par FACS. Les sous-populations de CD ont été définies par leurs marqueurs membranaires suivants : B220 and SyglecH pour les pCD , CD11c$^{high}$ and CD8$^{high}$ pour les cCD CD8+; CD11c$^{high}$ et CD8$^{low}$ pour les cCD CD8-. Expression des marqueurs de maturation des CD. Les marqueurs membranaires suivants ont été analysés par FACS: CMH-Classe II, CD80, CD86 et CD40.

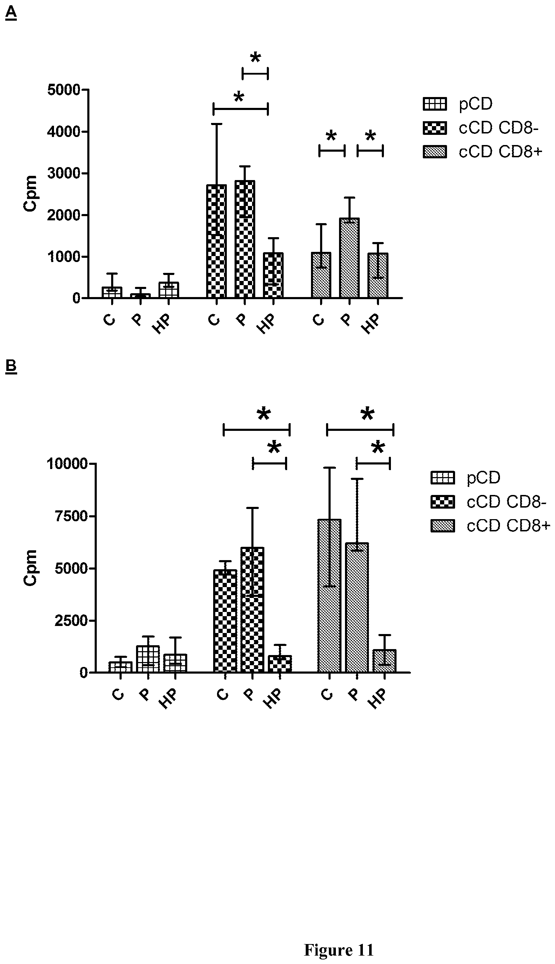

- Figure 11: L'IS post hémorragique diminue la capacité des cCD à faire proliférer des lymphocytes T durant une pneumopathie à SAMS.

Chaque sous-population de CD a été triée **(A.** CD non stimulées) et cultivée *ex vivo* pendant 24 heures avec du CpG 1826 (5$\mu$M): **(B.** CD stimulées). Chaque sous-population a été cultivée avec des lymphocytes T CD4 et CD8 allogéniques pendant 3 jours (ratio CD/T : 1/25). La prolifération lymphocytaire a été mesurée par l'incorporation cellulaire de thymidine sur une période de 8 heures. Chaque groupe comprenait un pool de 5 animaux. Les données ont été exprimées en médiane $\pm$ SEM et sont représentatives de deux expériences indépendantes, chacune ayant la même signification statistique. *pCD (barres quadrillées), cCD CD8- (barres à damier) et cCD CD8+ (barres grises).*

(* p ≤0.05).

> **A.** Prolifération des lymphocytes T en contact des CD non stimulées.
> **B.** Prolifération des lymphocytes T en contact des CD stimulées 24 heures par CpG.

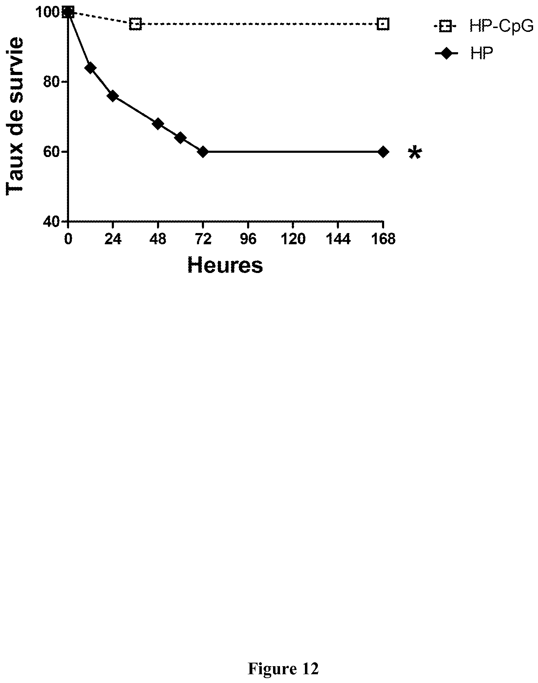

- Figure 12: Après choc hémorragique, un traitement par CpG-ODN prévient l'augmentation de susceptibilité des animaux à une pneumopathie à SASM.
  Une injection intra-veineuse de CpG-ODN 1668 (100μl à 100μmol) (groupe HP-CpG) ou du même volume de sérum salé isotonique (groupe HP) a été réalisée immédiatement après la réanimation du choc hémorragique. Vingt quatre heures plus tard, les souris ont été inoculées avec 7 x 10$^6$ CFU de SASM. Le taux de survie de chaque groupe a été mesuré deux fois par jour pendant 7 jours (168 heures). Chaque groupe comprenait 8 animaux. Les données ont été représentées en pourcentage et sont représentatives de trois expériences indépendantes, chacune ayant la même signification statistique. (* p ≤ 0.05). Groupe HP (losanges noires), groupe HP-CpG (carrés vides).

La présente invention sera mieux comprise à la lumière des exemples ci-dessous qui s'appuient sur les figures sus-présentées. Ces exemples sont fournis ici à titre purement illustratif et ne limitent en rien l'objet de la présente invention.

## EXEMPLES

### 1 Matériels et méthodes

#### 1.1. Animaux

Des souris mâles Balb/cJ (20-24 grammes) ont été obtenues auprès de l'élevage Janvier (Laval, France). Les souris ont été maintenues sur un cycle jour/nuit de 12 heures avec libre accès à l'eau et à la nourriture. Toutes les manipulations ont été conduites en accord avec les principes de soins des animaux de laboratoires (NIH publication N°86-23, modifiée en 1985). Trois groupes ont été définis: Contrôle (groupe C) consistant en une ponction cardiaque suivi d'une instillation stérile endo-trachéale ; Pneumopathie (groupe P) consistant en une pneumopathie seule ; et Hémorragie-Pneumopathie (groupe HP) consistant en un polytraumatisme (ici, un choc hémorragique) réanimé suivi d'une pneumopathie.

#### 1.2. Procédure de réalisation du choc hémorragique

Un modèle murin de choc hémorragique à volume contrôlé réanimé validé dans la littérature a été utilisé (Asehnoune et al. 2006 (A et B) ; Asehnoune et al. 2005; Robinson et al. 1991). Les animaux ont été anesthésiés avec 1.5 ml d'Isoflurane® (Baxter, France). Une ponction cardiaque trans-thoracique avec une aiguille de 29 gauges a été réalisée pour prélever sur 45 secondes 30% de la masse sanguine (0.3 ml de sang/10g de poids de corps). Le sang a été prélevé dans une seringue héparinée (heparinate de sodium, 5U) et conservé sous agitation à 37°C pendant 60 minutes. Le volume de sang a ensuite été restitué par injection dans le plexus veineux rétro-orbitaire.

#### 1.3. Procédure de réalisation de la pneumopathie à *Staphylococcus aureus*

Une souche de *Staphyloccocus aureus* methicilline sensible ou « SASM » (souche ATCC 29213) a été cultivée 16 heures à 37°C dans un milieu tryptic soja (Grosseron, Saint Herblain, France). Immédiatement avant utilisation, les cultures ont été lavées deux fois (centrifugées 10 minutes à 1000 g) et diluées dans du sérum salé isotonique stérile pour être calibrées par spectroscopie. La concentration bactérienne a systématiquement été contrôlée par culture quantitative.

Les souris ont été anesthésiées par Isoflurane® (Baxter, France), et placées en décubitus dorsal. Une aiguille de nutrition entérale (24 gauges) a été utilisée pour le cathétérisme de la trachée et l'injection de 70 μl de solution bactérienne. Les souris ont alors été suspendues 30 secondes par les incisives pour améliorer la pénétration de l'inoculum. Le taux d'instillation intra trachéale atteignait 100%.

#### 1.4. Surveillance Clinique

La mortalité a été évaluée deux fois par jours pendant 7 jours. Les souris ont été pesées une fois par jour pendant 5 jours. La glycémie capillaire a été mesurée par un haemaglucotest (Boehringer Ingelheim France). La gazométrie veineuse, reflet de l'intensité du choc et de la qualité de sa réanimation, a été mesurée sur sang veineux central prélevé dans l'oreillette droite.

**1.5. Mesure de la dissémination bactérienne de l'infection**

Après sacrifice, les poumons et la rate des animaux ont été homogénéisés mécaniquement de manière stérile. Les homogénats ont été soumis à une série de dilutions au 1/10ème et cultivés à 37°C sur milieu sélectif Chapman (Grosseron, Saint Herblain, France). Après 48 heures d'incubation, les colonies bactériennes ont été comptées et les résultats ont été exprimés en log10 Colonie Formant Unité (CFU) par gramme d'organe. Le staphylo slide reactif (Biorad, France) a été utilisé pour différencier *S. aureus* de *S.*non *aureus.*

**1.6. Analyse histologique**

Les poumons ont été prélevés et immédiatement placés dans du formol 4%. Les tissus fixés ont été découpés puis colorés par hématoxyline et éosine avant analyse microscopique.

**1.7. Mesure de l'activité myélopéroxydasique**

Comme précédemment décrit dans la littérature (Kim et al. 2005), après prélèvement, les poumons ont été pesés puis homogénéisés mécaniquement à +4°C pendant 25 secondes dans 1 ml de tampon (Phosphate de potassium à 50 mM avec du N-ethylmaleimide (10 mM), pH=6). L'homogénat protéique a été lavé deux fois dans le même tampon glacé. Avant sonication sur glace (180 secondes), le culot protéique a été mis en suspension dans 1 ml de phosphate de potassium (50 mM, pH=6) contenant 0.5% d'hexadecyl trimethylamonium. Après un choc thermique de 2 heures à +56°C, l'activité myélopéroxydasique du surnageant a été déterminée par mesure du pouvoir d'oxydation dépendant de l'eau oxygénée de l'o-ianiside (Kim et al. 2005).

**1.8. Mesure de la perméabilité endothéliale pulmonaire**

Comme précédemment décrit (Boutoille et al. 2009), l'albumine marquée à la Fluorescéine isothiocyanate (albumine FITC, Sigma, Allemagne) a été utilisée pour mesurer la perméabilité pulmonaire endothéliale. Deux heures après une injection intrapéritonéale de 2 mg d'albumine FITC, les souris ont été euthanasiées par exsanguination. Les poumons ont alors été prélevés, homogénéisés mécaniquement dans 1 ml de sérum salé isotonique puis centrifugés (4000g pendant 10 minutes). Le sang a été centrifugé 10 minutes à 4000g pour récupérer le plasma. La mesure de la quantité d'albumine FITC a été réalisée par fluorimétrie (excitation 487 nm et émission 520 nm) dans le plasma et le broyat pulmonaire La perméabilité endothéliale pulmonaire a alors été calculée selon la formule validée suivante :

$$\text{Perm-FITC (\%)} = (((FL_{HS} - FL_N) \times W_H) - QFB) / ((F_{BS} - F_{BN}) \times We \times 0.07 \times (1 - Hte))$$

où $FL_{HS}$ est la fluorescence du surnageant pulmonaire ; $FL_N$ est la fluorescence naturelle de ce surnageant mesurée sans albumine-FITC ; $F_{BS}$ est la fluorescence du plasma ; We x 0.07 x (1 - Hte) est le volume plamatique (We : poids de la souris ; Hte : hématocrite) ; $F_{BN}$ est la fluorescence naturelle du plasma mesurée sans administration d'albumine-FITC ; $W_H$ est le poids des poumons ; QFB, qui est la proportion de fluorescence correspondant au sang intrapulmonaire résiduel, est calculé ainsi :

$$(QFB = ((FL_{HS} - FL_N) \times Hb_{HS}) / Hb)$$

où $Hb_{HS}$ est le taux résiduel d'hémoglobine dans le surnageant pulmonaire et Hg est l'hémoglobinémie de la souris avant sacrifice.

**1.9. Préparation des homogénats pulmonaires pour dosage des cytokines par méthode ELISA**

Les poumons ont été homogénéisés dans un tampon glacé (1 X PBS, pH 7.4, 0.1 % Triton X-100) contenant 1 mM d'inhibiteur de protéases (Sigma, France) (24). Les surnageants obtenus après centrifugation à +4°C pendant 20 minutes à 12000g ont été conservés à -80°C jusqu'à analyse. La concentration protéique de chaque échantillon a été déterminée par kit de mesure protéique micro-BCA avec standardisation à l'albumine sérique bovine (Pierce, Angleterre).

### 1.10. Culture cellulaire pour mesure de la réactivité sanguine au LPS

Un échantillon de 0.5 ml de sang a été récolté lors du sacrifice des animaux. L'échantillon a été dilué au 1/5 dans du RPMI-1640 (Laboratoire de biotechnologies, Reims) avec supplémentation en pénicilline/streptomycine. Le sang dilué, 500 µL par puits, a été cultivé sans ou avec LPS (*Escherichia coli* O111:B4 à 1 µg/ml) à 37.0°C dans une étuve à 5% de $CO_2$ (20). Le surnageant des cultures cellulaires a été centrifugé à 12000g pendant 2 minutes et conservé à -80°C avant dosage ELISA. Les concentrations de TNFalpha, d'Interleukine (IL)-1β et de macrophage-inflammatory protein-2 (MIP-2) ont été quantifiées par ELISA selon les instructions du fournisseur (R&D Systems, France).

### 1.11. Isolement des cellules CD11c positives spléniques

Les rates ont été digérées par collagénase D (Roche Diagnostic, Allemagne) dans du RPMI 1640/1% sérum veau foetal pendant 25 minutes à 37°C. de l'EDTA à 10 mM a été ajouté pour les 5 dernières minutes avant filtration (80µm). Les cellules ont été lavées dans du PBS avant incubation 15 minutes à +4°C avec un anticorps anti-souris anti CD11c couplé à une bille métallique. Après lavage, la suspension cellulaire a été enrichie en cellules CD11c+ par sélection positive sur colonne de séparation MACS (Miltenyi, France). La pureté des cellules CD11c+ atteignait en routine 85 à 95%.

### 1.12. Réaction de polymérase en chaîne avec transcription inverse en temps réel (Real Time RT-PCR)

L'ARN total des cellules CD11 c+ spléniques a été isolé avec du TRIzol (Invitrogen, France) et traité 45 minutes à 37°C avec 2 U de RQ1 DNAse (Promega, France). Un microgramme d'ARN a subi une rétrotranscription avec de la Superscript III reverse transcriptase (Invitrogen). Un microlitre de solution d'ADN complémentaire a été soumis à une PCR quantitative en temps réel dans un système BioRad iCycler iQ utilisant le QuantiTect SYBR Green PCR kit (Qiagen, France). La PCR quantitative consistait en 45 cycles de 30 secondes à 95°C suivi de 30 secondes à 60°C. Les séquences des amorces pour le TNFalpha, l'Interferon beta (IFNbeta), l'IL-10, l'IL-12 et la glyceraldehyde-3-phosphate deshydrogenase (GAPDH) ont été sélectionnées grâce au logiciel "pick primer" de NCBI (tableau 1).

**Tableau 1: Séquences des amorces pour la RT-PCR quantitative**

| Amorce | Amorce forward (5'-3') | Amorce reverse (3'-5') |
|---|---|---|
| TNF alpha | *AAAGGGAGAGTGGTCAGGTTGC* | *GGCTGGCTCTGTGAGGAAGG* |
| IFN beta | *CCCTATGGAGATGACGGAGA* | *CTGTCTGCTGGTGGAGTTCA* |
| IL-10 | *TGGCATGAGGATCAGCAGGG* | *GGCAGTCCGCAGCTCTAGG* |
| IL-12p40 | *TGTGGAATGGCGTCTCTGTCTG* | *CAGTTCAATGGGCAGGGTCTCC* |
| GAPDH | *ACCACAGTCCATGCCATCAC* | *ACCTTGCCCACAGCCTTG* |

L'expression de GAPDH a été utilisée comme référence pour normaliser le niveau d'expression de chaque gène. L'expression génique quantitative a été calculée avec la méthode de 2-ΔΔCt en utilisant les cellules CD11c+ spléniques des souris Contrôle comme calibrateur (Livak et al. 2001).

### 1.13. Analyse par FACS

Le nombre total de cellules spléniques vivantes en suspension (PBS-Serum de Veau Foetal-Azide) a été compté sur cellules de Malassez en utilisant l'exclusion de l'éosine. Les anticorps anti-souris issus du rat utilisés ont été obtenus à partir de la collection européenne de culture cellulaire. Le mélange d'anticorps utilisé pour exclure les cellules non dendritiques était obtenu au près de BD biosciences (United States): CD3-PE, CD19-PE, TCRb-PE, NK1.1-PE, Ter119-PE . Les sous-populations des CD ont été caractérisées par les anticorps suivants : SiglecH-APC Biosciences (United States), CD11 c-PECy7, CD8-FITC, B220-PerCPCy5.5. La maturation des CD a été appréciée par les anticorps biotinylés suivants : anti-CD80, anti-CD86, anti-CD40 et anti-CMH de classe II. L'aspécificité des anticorps a été mesurée par l'ajout de Streptavidine-APC Cyanine 7 BD Biosciences (United States): Un total de $4 \times 10^6$ cellules pour chaque anticorps a été compté.

**1.14. Coculture lymphocytes T/cellules dendritiques (Ouabed et al. 2008)**

Les sous-populations de DC ont été triées par FACS et maturées (CD stimulées) ou non (CD non stimulées) 24 heures par du CpG 1668 (Invivogen, France). Les CD ont ensuite été cultivées avec des lymphocytes T CD4+ et CD8+ allogéniques dans une plaque de culture de 96 puits à fond rond. Après trois jours de culture à 37°C à 5% de $CO_2$, les cellules ont été mises 8 heures en contact avec $0.5\mu Ci$ de [3H]TdR (GE Healthcare) par puits. Les cellules ont alors été récoltées sur un filtre en fibres de verre pour mesure de l'incorporation de thymidine par scintillation (Packard Institute).

**1.15. Analyse statistique**

Le logiciel GraphPad prism (GraphPad Software, San Diego, California) a été utilisé pour réaliser les analyses statistiques. Les taux de survie ont été comparés par un test de logrank. Les variables continues non paramétriques ont été exprimées en médiane $\pm$ SEM ou $\pm$ 25-75ème percentiles et comparées par un test de Kruskall Wallis suivi d'un Mann-Whitney. $p \leq 0.05$ était le seuil de significativité statistique.

**2 Résultats**

**2.1. Etude pilote : mise en place d'un modèle murin d'immunodépression systémique (IS) post hémorragique secondairement exposé à une pneumopathie à S. _aureus_.**

_2.1.1 Effet de l'inoculum et du délai entre choc hémorragique et pneumopathie à SASM sur la mortalité_

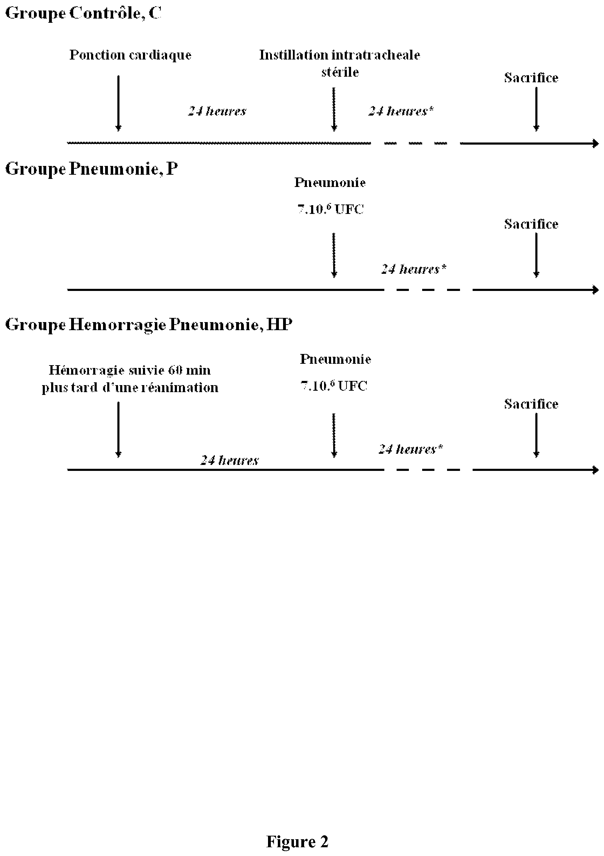

Dans le but de mettre en évidence les effets de l'IS post hémorragique sur la mortalité, une pneumopathie à SASM a été réalisée avec $7.10^5$, $7.10^6$ and $7.10^7$ UFC de _S. aureus_ 24 heures après réalisation d'un choc hémorragique (groupe HP) et comparée avec la réalisation d'une pneumopathie seule (groupe P). La mortalité était supérieure dans le groupe HP comparée au groupe P pour les 2 inocula les plus faibles (Figure 1.A.B). L'inoculum supérieur entraînaît une mortalité de 100% dans les deux groupes (Figure 1.C). Comme l'IS post hémorragique est un processus dynamique, l'évolution dans le temps de l'IS a été évaluée. Une pneumopathie à SASM a été réalisée 2, 4, 8, 24, 48 ou 96 heures après le choc hémorragique (groupe HP-H2 ; -H4 ; -H8 ; H-24 ; H-48 ; H-96, respectivement). Comparée à une pneumopathie seule (groupe P), le taux de survie dans le groupe HP diminuait significativement avec l'augmentation du délai entre choc et pneumopathie de 4 à 24 heures, et une récupération progressive était observée pour des durées supérieures (Figure 1.D). Un inoculum de _S. aureus_ de $7.10^6$ UFC (groupe P) et un délai entre le choc hémorragique et la pneumopathie de 24 heures (groupe HP) étaient les paramètres optima pour l'étude de l'IS. Ils ont donc été utilisés pour l'étude principale (Figure 2). Dans le groupe Contrôle (C), une ponction cardiaque sans prélèvement sanguin ni réanimation était suivie d'une instillation intra-trachéale de sérum salé stérile 24 heures plus tard. L'hémorragie à volume contrôlée a été réalisée par ponction cardiaque (0.3 ml/10g) suivi d'une réinjection du sang prélevé 60 minutes plus tard (groupe Hémorragie Pneumopathie, HP). Vingt quatre heures après la réanimation, 70 $\mu$l soit de $7.10^6$ UFC de SASM (groupe Pneumopathie, P et groupe HP) soit de sérum physiologique stérile (groupe C) ont été instillés dans la trachée. Vingt quatre heures (sauf si précisé autrement) après le début de l'infection, les souris ont été euthanasiées et les prélèvements réalisés.

_2.1.2 Le choc hémorragique aggrave la perte de poids et les conséquences biologiques d'une pneumopathie à SASM._

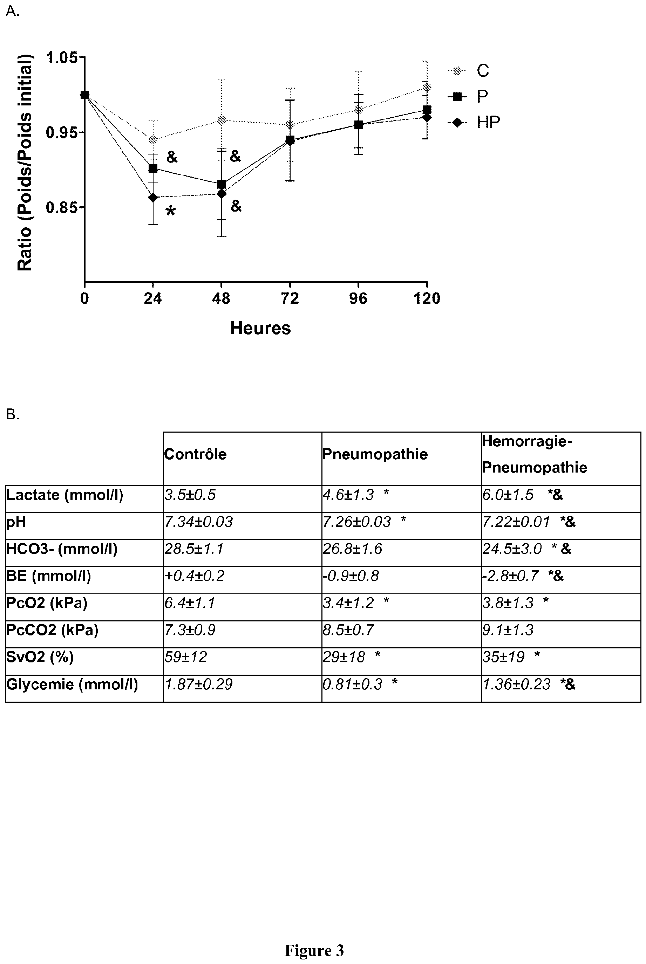

Les conséquences cliniques et biologiques de l'IS post hémorragique sur le devenir de l'infection ont également été mesurées. Les souris du groupe HP ont subi une plus grande perte de poids (Figure 3.A), une acidose métabolique plus sévère et une chute moins importante de la glycémie (Figure 3.B) que celles du groupe P.

_2.1.3. L'IS post hémorragique induit des bactériémies au cours d'une pneumopathie à SASM._

La dissémination locale et systémique de l'infection a été évaluée 12, 24 et 48 heures après la pneumopathie avec (groupe HP) et sans choc hémorragique (groupe P). Aucun SASM n'a été mis en évidence dans les poumons et la rate des souris contrôles (groupe C). La clairance pulmonaire bactérienne était inchangée dans le groupe HP en comparaison au groupe P (Figure 4.A). En revanche, la dissémination systémique de l'infection, évaluée par les cultures des broyats de rates, augmentait en intensité à la 24ème et 48ème heure dans le groupe HP comparé au groupe P (Figure 4.B). Les bactériémies étaient également plus fréquentes après choc hémorragique aux 24ème et 48ème de l'infection (Figure 4.C).

*2.1.4. L'IS post hémorragique aggrave les lésions pulmonaires d'une pneumopathie à SASM.*

La clairance pulmonaire bactérienne n'étant pas altérée au cours de l'IS post hémorragique, l'aspect histo-pathologique des poumons au cours de l'infection a été évalué. Le tissu pulmonaire sain est caractérisé par de fines alvéoles aérées bordées d'une couche unicellulaire de pneumocytes. (Figure.5.A). Pour les souris contrôles (groupe C), le tissu pulmonaire apparaissait normal (Figure 5.B). Dans le groupe P, dès les 12ème et 24ème heures de l'infection, on observait des agrégats de cellules immunitaires et un début de destruction alvéolaire avec épaississement des parois (figure 5.C.E). La récupération histologique avec une aération des alvéoles débutait au 4ème jour et était complète au 7ème jour (Figure 5.G.I). Dans le groupe HP, à chaque temps (Figure 5.D.F.H.J), les agrégats de cellules immunitaires étaient plus nombreux et les lésions alvéolaires plus sévères que dans le groupe P. Les lésions pulmonaires apparaissaient plus rapidement et la récupération était plus prolongée dans le groupe HP que dans le groupe P.

Les lésions histologiques d'une pneumopathie à SASM étaient qualitativement plus sévères après un choc hémorragique, avec notamment une augmentation de l'afflux les cellules immunes (macrophages et polynucléaires neutrophiles). L'accumulation pulmonaire de polynucléaires neutrophiles a donc été évaluée quantitativement en dosant l'activité myélopéroxydasique du tissu pulmonaire des souris. A la 24ème heure de l'infection, l'accumulation de polynucléaires neutrophiles était plus importante dans le groupe HP que dans le groupe P (Figure 6.A). Les polynucléaires produisent des espèces radicalaires de l'oxygène en grande quantité, celles-ci sont source de lésions endothéliales (Rivkind et al. 1991). Du fait de l'accumulation de ces cellules, la perméabilité endothéliale pulmonaire (reflet de l'oedème alvéolaire) a été mesurée. Elle s'élevait dans le groupe HP de manière plus importante que dans le groupe P (Figure 6.B). Par ailleurs, les concentrations pulmonaires en cytokines pro-inflammatoires (TNF alpha, IL-1 beta et MIP-2) étaient augmentées au cours de l'infection (Figure 6.C.D.E) mais celle de l'IL-1 beta s'élèvait plus quand la pneumopathie était précédée du choc hémorragique (Figure 6.D).

*2.1.5. L'IS post hémorragique majore l'hypo-réactivité sanguine au LPS au décours d'une pneumopathie à SASM*

Comme les Inventeurs (Asehnoune et al. 2006A) et d'autres auteurs (Goebel et al. 2000 ; Spolarics et al. 2003) l'avaient précédemment décrit, l'IS post traumatique est caractérisée par une baisse de la réactivité sanguine au LPS d'*E.coli O111B4*. Puisque l'étude de la sécrétion de cytokines sur sang total produit les mêmes résultats que sur monocytes isolés (Damsgaard et al. 2009), les Inventeurs ont choisi, pour des raisons techniques liées à leur modèle murin, d'étudier l'hyporéactivité au LPS sur sang total. La production de TNF alpha, d'IL-1 beta était significativement diminuée dans le groupe HP en comparaison aux groupes P et S (Figure 7.A.B.) alors que celle de MIP-2 était également diminuée dans les groupes HP et P versus le Groupe C (Figure 7.C.).

**2.2. Caractérisation des troubles de l'immunité innée liées à l'IS post hémorragique au décours d'une pneumopathie à SASM.**

Lors de la réponse de l'organisme à une infection, les cellules dendritiques assurent l'intégration entre immunité innée et adaptative et ont probablement un rôle crucial dans la régulation de la dysfonction immune induite par l'hémorragie. La diminution de l'expression membranaire de HLA-DR sur les monocytes circulants est pour l'instant le seul marqueur d'immunodépression systémique à avoir donné des résultats concordants en termes de prédiction de la survenue d'une infection, d'un syndrome e défaillance multiviscérale et de corrélation au pronostic post-traumatique en général sans qu'aucun lien de causalité n'ait été démontré. Dans le modèle d'IS post hémorragique ici décrit, l'infection est plus grave lorsqu'elle est précédée par une hémorragie. Le nombre et la fonction des cellules dendritiques ont donc été comparés en fonction de la présence (groupe HP) ou non (groupe P) d'un choc hémorragique avant l'infection.

*2.2.1. Nombre et maturation des cellules CD11c^high spléniques au décours d'une pneumopathie à SASM précédée ou non d'un choc hémorragique.*

Le nombre de cellules CD11c^high, qui représentent la majorité des CD dans la rate, était diminué au décours de l'infection qu'elle soit (groupe HP) ou non (groupe P) précédée d'un choc hémorragique (Figure 8.A.). Les capacités de présentation de l'antigène ont été mesurées par l'expression membranaire des molécules de présentation de l'antigène (CMH de Classe 2), et des molécules de costimulation (CD80 et le CD86). Les expressions membranaires du CMH de classe 2 et de CD80 étaient diminuées durant la pneumopathie à SASM dans les deux groupes P et HP, alors que l'expression membranaire de CD86 n'était diminuée que lorsqu'un choc hémorragique était réalisé avant le début de l'infection (Figure 8.B.). L'expression membranaire de CD40, marqueur de la maturation des CD, n'était pas modifiée dans les conditions de l'expérience (Figure 8.B.).

*2.2.2. L'IS post hémorragique diminue l'activité transcriptionnelle des cellules CD11c[+] au décours d'une pneumopathie à SASM.*

Les cellules CD11c[+] comprennent l'ensemble des CD murines. Les taux d'ARNm de TNF alpha, d'IFN beta et d'IL-12 étaient significativement élevés à la 6ème heure de l'infection dans le groupe P en comparaison au groupe C (Figure 9.A.B.C.) Cette augmentation n'était pas observée lorsque l'infection était précédée d'un choc hémorragique (groupe HP) (Figure 9.A.B.C.). Pour l'IL-10, le niveau d'ARNm chutait dans le groupe HP en comparaison au groupe C alors qu'il était inchangé en cas d'infection seule (Figure 9.D.).

2.2.3. L'IS post hémorragique altère les phénotypes des sous populations de CD au cours d'une pneumopathie à SASM.

Trois sous-populations principales de CD sont à ce jour décrites (Merad et al. 2009) : les CD conventionnelles (cCD) qui comprennent les cCD CD8+ et les cCD CD8- ; les CD plasmacytoïdes (pCD). Le nombre de cCD CD8- était diminué 24 heures après la pneumopathie, qu'elle soit (groupe HP) ou non (groupe P) précédée d'un choc hémorragique alors que les nombres de cCD CD8+ et de pCD étaient inchangés (Figure 10.A). Concernant les pCD, l'hémorragie réalisée avant la pneumopathie (groupe HP) entraînait une diminution significative des capacités de présentation de l'antigène (expression membranaire du CMH-Classe II, CD80 et CD86) comparé à la réalisation d'une pneumopathie seule (groupe P) et du groupe contrôle (C). La maturation des pCD, reflétée par l'expression de CD40, était également diminuée par l'infection avec (groupe HP) ou sans (groupe P) choc hémorragique comparé au groupe contrôle (groupe C) (Figure 10.B). Concernant les cCD CD8+, la maturation et les capacités de présentation de l'antigène étaient toutes les deux diminuées par l'infection avec (groupe HP) ou sans (groupe P) choc hémorragique comparé au groupe contrôle (groupe C, Figure 10.C). Concernant les cCD CD8-, les capacités de présentation de l'antigène étaient partiellement diminuées (expression membranaire des CMH-classe II et CD80) alors que la maturation (expression membranaire du CD40) était inchangée dans les animaux infectés après (groupe HP) ou sans (groupe P) choc hémorragique comparé aux animaux contrôles (groupe C, Figure 10.D).

*2.2.4. L'IS post hémorragique diminue la capacité des cCD à faire proliférer des lymphocytes T durant une pneumopathie à SAMS*

L'effondrement des marqueurs membranaires étudiés par FACS lors d'une pneumopathie à MSSA (groupe P) était tellement important dans le modèle ici décrit que les possibilités techniques de mise en évidence d'une différence au cours de l'IS post hémorragique étaient limitées. Pour affiner la compréhension des troubles fonctionnels des CD après choc hémorragique, les capacités de chaque sous-population de CD à entraîner la prolifération des lymphocytes ont été étudiées sans (Figure 11.A.) et avec (Figure 11.B.) maturation *ex vivo* des CD par CpG. Le choc hémorragique (groupe HP) diminuait drastiquement les capacités des cCD (CD8+ et CD8-) à faire proliférer des lymphocytes T, avec et sans maturation par CpG comparé à une pneumopathie seule (groupe P) (Figure 11.A.B.). Aucune différence n'était apparente dans la sous-population des pCD (Figure 11.A.B.).

**2.3. Effet d'un traitement préventif par CPG-ODN ou par MPLA sur la surmortalité d'une pneumopathie à SASM dans notre modèle d'IS post hémorragique.**

Puisque les trois sous-populations de CD étaient significativement altérées par l'IS post hémorragique, les Inventeurs ont testé la possibilité de prévenir la surmortalité liée à ce trouble durant une pneumopathie à MSSA par un traitement par agoniste du TLR-9, le CpG-ODN qui déclenche *in vivo* la maturation des CD en conditions normales (Askew et al. 2000) ou par un agoniste du TLR-4 , le MPLA qui déclenche la maturation des CD conventionnelles *in vivo* (Mata-Haro et al. 2007). Le CpG 1668 (CpG-ODN de type B, Invivogen, France), tout comme le MPLA (Invivogen, France), diminuait significativement la mortalité dans le modèle ici décrit (Figure 12 et données non montrées). Le CpG-ODN inactif (contrôle), qui est un agoniste du TLR-9 n'activant pas son récepteur, ne prévenait pas la mortalité liée à l'infection.

**3 Observations complémentaires**

Les résultats présentés plus haut confirment le rôle critique joué par les altérations fonctionnelles des CD dans les infections secondaires à l'IS post hémorragique. Il est également montré que 1) l'IS post hémorragique augmente la mortalité, la dissémination systémique et les lésions pulmonaires d'une pneumopathie à *S. aureus ; 2)* les principales sous populations de CD sont altérées dans ce trouble ; 3) un traitement préventif par CpG injecté lors de la phase de réanimation du choc hémorragique permet de corriger la surmortalité d'une pneumopathie à *S. aureus* liée à l'IS post hémorragique.

**REFERENCES**

Mathers et al. PloS Med. 2006 Nov; 3(11):e442.
Patton et al. Lancet. 2009 Sep;374(9693):881-92.
Rincon-Ferrari et al. J Trauma. 2004 Dec;57(6):1234-40.
Papia et al. J Trauma. 1999 Nov;47(5):923-7.
SFAR, SRLF. The risk for and approaches to control nosocomial ionfections in ICUs: guidelines from SFAR/SRLF task force on nosocomial infections in ICUs reanimaiton. 2005;14:463-71.
Keel et al. J Trauma. 1996 Sep;41(3):430-7; discussion 7-8.
Ditschkowski et al. Ann Surg. 1999 Feb;229(2):246-54.
Stephan et al. J Surg Res. 1989 Jun;46(6):553-6.
Wichmann et al. Crit Care Med. 1998 Aug;26(8):1372-8.
Asehnoune et al. Resuscitation. 2006A Jan;68(1):127-33.
Asehnoune et al. Cytokine. 2006B May;34(3-4):212-8.
Asehnoune et al. Ann Fr Anesth Reanim. 2005 Mar;24(3):255-9.
Robinson et al. Crit Care Med. 1991 Oct;19(10):1285-93.
Kim et al. Am J Physiol Lung Cell Mol Physiol. 2005 May;288(5):L958-65.
Boutoille et al. Exp Lung Res. 2009 May;35(4):263-71.
Livak et al. Method. Methods. 2001 Dec;25(4):402-8.
Ouabed et al. J Immunol. 2008 May 1;180(9):5862-70.
Rivkind et al. Circ Shock. 1991 Jan;33(1):48-62.
Goebel et al. Ann Surg. 2000 Feb;231 (2):253-61.
Spolarics et al. Crit Care Med. 2003 Jun;31(6):1722-9.
Damsgaard et al. J Immunol Methods. 2009 Jan 30;340(2):95-101.
Merad et al. Blood. 2009 Apr 9;113(15):3418-27.
Askew et al. J Immunol. 2000 Dec 15;165(12):6889-95.
Mata-Haro et al. (2007) Science 316: 1628-1632.
Léonard et al. (2007) Clin. Cancer Res. 13:6168-6174.
Venet et al. (2007) Crit. Care Med. 35 :1910-1917.
Cheadle et al. (1991) Am. J. Surg. 161 :639-645.
Baker et al. (1974) J Trauma. 14 :187-196.
Teasdale et al. (1974) Lancet 2: 81-84.
Osborn et al. Crit Care Med 2004:32(11):2234-40.

**Revendications**

1.  Composition pharmaceutique comprenant au moins un agoniste d'au du récepteur de type Toll 4 (TLR 4), pour son utilisation dans le traitement prophylactique des complications septiques de l'immunodépression systémique post-traumatique chez un patient hospitalisé atteint d'un ou plusieurs traumatismes sévères, ledit agoniste étant choisi parmi le lipide A monophosphoryle (MPLA) et le lipide A monophosphoryle 3-O désacylaté (3D-MPLA).

2.  Composition selon la revendication 1, **caractérisée en ce que** ladite immunodépression se **caractérise par** :

a) une baisse du niveau de production *ex vivo* de cytokines pro-inflammatoires induites par des leucocytes sanguins après stimulation par du LPS de bacilles gram négatif, par rapport au niveau de production observé pour un individu sain ; et/ou

b) une baisse du niveau d'expression de HLA-DR sur les cellules présentatrices des antigènes dudit patient, par rapport au niveau d'expression observé pour un individu sain.

**3.** Composition selon la revendication 2, **caractérisée en ce que** le niveau d'expression de HLA-DR sur les monocytes dudit patient dans les 24 heures suivant le ou les traumatismes est diminué par rapport au niveau d'expression observé pour un individu sain.

**4.** Composition selon l'une quelconque des revendications 1 à 3, **caractérisée en ce que** lesdites complications septiques sont des infections nosocomiales, de préférence bactériennes.

**5.** Composition selon l'une quelconque des revendications 1 à 4, **caractérisée en ce que** ledit patient traumatisé présente au moins deux lésions traumatiques parmi lesquelles au moins une lésion engage le pronostic vital.

**6.** Composition selon la revendication 5, **caractérisée en ce que** ledit patient traumatisé présente un score de gravité des lésions (ISS) d'au moins 16 et/ou un traumatisme crânien sévère défini par un score de Glasgow (CGS) inférieur à 8.

**Patentansprüche**

**1.** Pharmazeutische Zusammensetzung, welche mindestens einen Agonisten des Toll-like Rezeptors 4 (TLR-4) umfasst, zur Verwendung in der prophylaktischen Behandlung von septischen Komplikationen der posttraumatischen systemischen Immundepression bei einem hospitalisiert Patienten, der ein oder mehrere schwere Traumata erlitten hat, wobei der besagte Agonist aus dem Monophosphoryllipid A (MPLA) und dem 3-0-desacyliertem Monophosphoryllipid A (3D-MPLA) ausgewählt ist.

**2.** Zusammensetzung nach Anspruch 1, **dadurch gekennzeichnet, dass** die besagte Immundepression **gekennzeichnet ist durch**:

a) einen Rückgang des ex vivo-Produktionsniveaus von proinflammatorischen Zytokinen, die von Leukozyten im Blut nach LPS-Stimulation von gramnegativen Bazillen induziert werden, im Vergleich zu dem bei einer gesunden Person beobachteten Produktionsniveau; und/oder

b) einen Rückgang des Expressionsniveaus von HLA-DR auf den die Antigene aufweisenden Zellen des besagten Patienten, im Vergleich zu dem bei einer gesunden Person beobachteten Expressionsniveau.

**3.** Zusammensetzung nach Anspruch 2, **dadurch gekennzeichnet, dass** das Expressionsniveau von HLA-DR auf den Monozyten des besagten Patienten in den 24 Stunden nach dem oder den Traumata im Vergleich zu dem bei einer gesunden Person beobachteten Expressionsniveau verringert ist.

**4.** Zusammensetzung nach einem der Ansprüche 1 bis 3, **dadurch gekennzeichnet, dass** die besagten septischen Komplikationen nosokomikale, bevorzugt bakterielle Infektionen sind.

**5.** Zusammensetzung nach einem der Ansprüche 1 bis 4, **dadurch gekennzeichnet, dass** der besagte Traumapatient mindestens zwei traumatische Läsionen aufweist, von denen mindestens eine Läsion lebensbedrohlich ist.

**6.** Zusammensetzung nach Anspruch 5, **dadurch gekennzeichnet, dass** der besagte Traumapatient einen Verletzungsschweregrad (ISS) von mindestens 16 und/oder ein schweres Schädeltrauma erlitten hat, das durch einen Glasgow-Score (GCS) von kleiner 8 definiert ist.

**Claims**

**1.** Pharmaceutical composition comprising at least one agonist of the receptor of the Toll 4 (TLR4) type, for use in the prophylactic treatment of septic complications of post-traumatic systemic immunodepression in a hospitalised patient suffering from one or more severe traumas, said agonist being chosen from monophosphoryl lipid A (MPLA) and 3-0-deacylated monophosphoryl lipid A (3D-MPLA).

2. Composition according to claim 1, wherein said immunodepression is **characterised by**:

   a) a reduction in the level of ex *vivo* production of pro-inflammatory cytokines caused by white blood corpuscles after stimulation by LPS from gram-negative bacilli, compared with the production observed for a healthy individual; and/or
   b) a reduction in the level of expression of HLA-DR on cells presenting antigens of said patient, compared with the expression level observed for a healthy individual.

3. Composition according to claim 2, wherein the level of expression of HLA-DR on the monocytes of said patient in the 24 hours following the trauma or traumas is reduced compared with the expression level observed for a healthy individual.

4. Composition according to any of claims 1 to 3, wherein said septic complications are nosocomial infections, preferable bacterial.

5. Composition according to any of claims 1 to 4, wherein said traumatised patient has at least two traumatic lesions, among which at least one lesion involving a vital prognosis.

6. Composition according to claim 5, wherein said traumatised patient has an injury severity score (ISS) of at least 16 and/or a severe cranial trauma defined by a Glasgow score (GCS) inferior to 8.

Figure 1

**Groupe Contrôle, C**

Ponction cardiaque

Instillation intratracheale
stérile

Sacrifice

*24 heures*

*24 heures\**

**Groupe Pneumonie, P**

Pneumonie

7.10.$^6$ UFC

Sacrifice

*24 heures\**

**Groupe Hemorragie Pneumonie, HP**

Hémorragie suivie 60 min
plus tard d'une réanimation

Pneumonie

7.10.$^6$ UFC

Sacrifice

*24 heures*

*24 heures\**

**Figure 2**

A.

B.

|  | Contrôle | Pneumopathie | Hemorragie-Pneumopathie |
|---|---|---|---|
| Lactate (mmol/l) | 3.5±0.5 | 4.6±1.3 * | 6.0±1.5 *& |
| pH | 7.34±0.03 | 7.26±0.03 * | 7.22±0.01 *& |
| HCO3- (mmol/l) | 28.5±1.1 | 26.8±1.6 | 24.5±3.0 * & |
| BE (mmol/l) | +0.4±0.2 | -0.9±0.8 | -2.8±0.7 *& |
| PcO2 (kPa) | 6.4±1.1 | 3.4±1.2 * | 3.8±1.3 * |
| PcCO2 (kPa) | 7.3±0.9 | 8.5±0.7 | 9.1±1.3 |
| SvO2 (%) | 59±12 | 29±18 * | 35±19 * |
| Glycemie (mmol/l) | 1.87±0.29 | 0.81±0.3 * | 1.36±0.23 *& |

Figure 3

**Figure 4**

**Figure 7**

**Figure 5**

**Figure 6**

A.

B.

**Figure 8**

**Figure 9**

**Figure 10**

Figure 11

Figure 12

**EP 2 519 259 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0971739 A
- EP 0772619 A
- WO 2009088401 A
- FR 0906372

**Littérature non-brevet citée dans la description**

- **MATHERS et al.** *PloS Med.,* Novembre 2006, vol. 3 (11), e442
- **PATTON et al.** *Lancet.,* Septembre 2009, vol. 374 (9693), 881-92
- **RINCON-FERRARI et al.** *J Trauma.,* Décembre 2004, vol. 57 (6), 1234-40
- **PAPIA et al.** *J Trauma.,* Novembre 1999, vol. 47 (5), 923-7
- The risk for and approaches to control nosocomial ionfections in ICUs: guidelines from SFAR/SRLF task force on nosocomial infections. *ICUs reanimaiton,* 2005, vol. 14, 463-71
- **KEEL et al.** *J Trauma.,* Septembre 1996, vol. 41 (3), 430-7
- **DITSCHKOWSKI et al.** *Ann Surg.,* Février 1999, vol. 229 (2), 246-54
- **STEPHAN et al.** *J Surg Res.,* Juin 1989, vol. 46 (6), 553-6
- **WICHMANN et al.** *Crit Care Med.,* Août 1998, vol. 26 (8), 1372-8
- **ASEHNOUNE et al.** *Resuscitation,* 2006, vol. 68 (1), 127-33
- **ASEHNOUNE et al.** *Cytokine,* 2006, vol. 34 (3-4), 212-8
- **ASEHNOUNE et al.** *Ann Fr Anesth Reanim.,* Mars 2005, vol. 24 (3), 255-9
- **ROBINSON et al.** *Crit Care Med.,* Octobre 1991, vol. 19 (10), 1285-93
- **KIM et al.** *Am J Physiol Lung Cell Mol Physiol.,* Mai 2005, vol. 288 (5), L958-65
- **BOUTOILLE et al.** *Exp Lung Res.,* Mai 2009, vol. 35 (4), 263-71
- **LIVAK et al.** *Method. Methods.,* Décembre 2001, vol. 25 (4), 402-8
- **OUABED et al.** *J Immunol.,* 01 Mai 2008, vol. 180 (9), 5862-70
- **RIVKIND et al.** *Circ Shock.,* Janvier 1991, vol. 33 (1), 48-62
- **GOEBEL et al.** *Ann Surg.,* Février 2000, vol. 231 (2), 253-61
- **SPOLARICS ; 2003 JUN et al.** *Crit Care Med.,* vol. 31 (6), 1722-9
- **DAMSGAARD et al.** *J Immunol Methods.,* 30 Janvier 2009, vol. 340 (2), 95-101
- **MERAD et al.** *Blood,* 09 Avril 2009, vol. 113 (15), 3418-27
- **ASKEW et al.** *J Immunol.,* 15 Décembre 2000, vol. 165 (12), 6889-95
- **MATA-HARO et al.** *Science,* 2007, vol. 316, 1628-1632
- **LÉONARD et al.** *Clin. Cancer Res.,* 2007, vol. 13, 6168-6174
- **VENET et al.** *Crit. Care Med.,* 2007, vol. 35, 1910-1917
- **CHEADLE et al.** *Am. J. Surg.,* 1991, vol. 161, 639-645
- **BAKER et al.** *J Trauma.,* 1974, vol. 14, 187-196
- **TEASDALE et al.** *Lancet,* 1974, vol. 2, 81-84
- **OSBORN et al.** *Crit Care Med,* 2004, vol. 32 (11), 2234-40